# EUROPEAN PATENT APPLICATION

(11) **EP 4 650 448 A1**
(43) Date of publication of application: **19.11.2025**
(21) Application number: 24791700.8
(22) Date of filing: 30.01.2024
(51) Int. Cl.: C12N 15/113, C12N 15/11, C12N 15/63, C12N 5/10, A61K 48/00

(54) **RNA ENZYME-BASED DEAR NUCLEIC ACID MANIPULATION SYSTEM AND USE THEREOF**

(30) Priority: 19.04.2023 CN 202310424082; 20.06.2023 WO PCT/CN2023/101437
(71) Applicant: Tsinghua University, Haidian District, Beijing 100084 (CN)
(72) Inventor: LIU, Junjie, Beijing 100084 (CN); LIU, Zixian, Beijing 100084 (CN); ZHANG, Shouyue, Beijing 100084 (CN); CHEN, Zhihang, Beijing 100084 (CN)
(74) Representative: Fuchs Patentanwälte Partnerschaft mbB
(86) International application number: PCT/CN2024/074724
(87) International publication number: WO 2024/217112

(57) **Abstract**

Disclosed in the present disclosure are an RNA enzyme-based DEAR nucleic acid manipulation system and a use thereof. The present disclosure provides a DEAR nucleic acid manipulation system, wherein the DEAR nucleic acid manipulation system comprises an RNA molecule derived from a bacterial group II intron of type C, and the RNA molecule contains a substrate recognition region hybridized with a target sequence in a target nucleic acid. The RNA enzyme-based DEAR nucleic acid manipulation system provided by the present disclosure avoids the problem that the transfection efficiency is affected by large protein molecules in a CRISPR-Cas system, the problem of the potential immunogenicity caused by Cas proteins, etc. The RNA enzyme-based DEAR nucleic acid manipulation system provided by the present disclosure can realize cutting of DNA and RNA.

## Description

### CROSS-REFERENCE TO RELATED APPLICATIONS

The present disclosure claims priority to Chinese patent application 202310424082.1 filed on April 19, 2023 and entitled "RNA RIBOZYME-BASED DEAR NUCLEIC ACID MANIPULATION SYSTEM AND USE THEREOF", and PCT international application PCT/CN2023/101437 filed on June 20, 2023 and entitled "RNA RIBOZYME-BASED DEAR NUCLEIC ACID MANIPULATION SYSTEM AND USE THEREOF", which are incorporated herein by reference in their entirety, including the Appendix.

### TECHNICAL FIELD

The present disclosure belongs to the field of biotechnology, and specifically relates to an RNA ribozyme-based DEAR nucleic acid manipulation system and use thereof, and more specifically to a programmable DEAR nucleic acid manipulation system with broad-spectrum cleavage capabilities for RNA and DNA as well as gene editing capabilities.

### BACKGROUND ART

Currently, the commonly used gene editing technologies in China are all developed based on the RNA-guided CRISPR-Cas nucleases, the core patents of which are owned by European and American countries. China has insufficient original innovation in gene editing technologies and lacks core patents.

However, there are still some problems with the CRISPR-Cas system. Firstly, the CRISPR-Cas system has an off-target effect, and editing by the Cas protein in non-target regions may cause uncontrollable deleterious variations. Secondly, the CRISPR-Cas system has the problem of excessively large protein size. The protein sizes of the currently mainly used CRISPR-Cas editing tool molecules, SpyCas9 and AsCas12a, both exceed 1300 amino acids. The excessively large molecular weights affect the transfection efficiency of the CRISPR-Cas system tools. Moreover, the CRISPR-Cas system may potentially cause immune responses. The currently used SpyCas9 and AsCas12a proteins are derived from pathogenic bacteria that humans have been exposed to, which may trigger immune responses in the human body.

Therefore, the CRISPR-Cas nuclease system is limited by the limitations of its protein elements. If a new generation of nucleic acid-targeting manipulation technology that is entirely based on RNA and simultaneously possesses both gene sequence-specific targeting and catalytic activity can be developed, it is expected to overcome the limitations of the application of the protease-based gene editing system.

### SUMMARY OF THE INVENTION

### Problems to be Solved by the Invention

Based on the various problems with the CRISPR-Cas nuclease system in the prior art, the object of the present disclosure is to provide an RNA ribozyme-based DEAR nucleic acid manipulation system, and apply it to the targeted modification (e.g., cleavage) of nucleic acids (DNA and RNA).

### Solutions for Solving the Problems

A first aspect of the present disclosure provides a DEAR nucleic acid manipulation system, wherein the DEAR nucleic acid manipulation system comprises an RNA molecule derived from a bacterial Group IIC intron, and the RNA molecule comprises a target recognition site that hybridizes to a target sequence in a target nucleic acid.

In some embodiments, the DEAR nucleic acid manipulation system comprises at least one of Domain I, Domain II, Domain III, Domain IV, Domain V, and Domain VI.

In some preferred embodiments, the DEAR nucleic acid manipulation system comprises at least Domain I, Domain II, Domain III, and Domain V.

In some specific embodiments, the Group IIC intron consists of Domains I-VI, in which each domain is in the form of a stem-loop structure and separated from each other, and the target recognition site is located in the top loop region of Domain I.

In some embodiments, the DEAR nucleic acid manipulation system is in the range of 100-5660 nt, preferably 124-3897 nt, in length.

In some embodiments, the Domain I comprises 2-6 stem-loop/hairpin structures in the range of 50-400 nt in length; and preferably, the Domain I comprises 3-5 stem-loop/hairpin structures in the range of 65-384 nt in length.

In some embodiments, the Domain II comprises 1-4 stem-loop/hairpin structures in the range of 10-300 nt in length; and preferably, the Domain II comprises 1-3 stem-loop/hairpin structures in the range of 10-218 nt in length.

In some embodiments, the Domain III comprises 1-3 stem-loop/hairpin structures in the range of 10-200 nt in length; and preferably, the Domain III comprises 1-2 stem-loop/hairpin structures in the range of 10-140 nt in length.

In some embodiments, the Domain IV comprises 0-4 stem-loop/hairpin structures in the range of 0-4500 nt in length; and preferably, the Domain IV comprises 0-4 stem-loop/hairpin structures in the range of 0-3000 nt in length.

In some embodiments, the Domain V comprises 1 stem-loop/hairpin structure in the range of 20-60 nt in length; and preferably, the Domain V comprises 1 stem-loop/hairpin structure in the range of 29-43 nt in length.

In some embodiments, the Domain VI comprises 1 stem-loop/hairpin structure in the range of 10-200 nt in length; and preferably, the Domain VI comprises 1 stem-loop/hairpin structure in the range of 10-112 nt in length.

In some embodiments, the Group IIC intron is a Group IIC intron in which an open reading frame encoding an intron-encoded protein is present or absent in Domain IV.

In some optional embodiments, the open reading frame encoding an intron-encoded protein is 0-4000 nt in length. In some embodiments, the target recognition site is located in the Domain I.

In some embodiments, the nucleotide sequence of the RNA molecule is selected from any one of:
(i) the nucleotide sequence comprises a nucleotide sequence as set forth in any one of the sequences SEQ ID NOs: 1-9 and 56;
(ii) the nucleotide sequence comprises a nucleotide sequence that is the reverse complement of a sequence as set forth in any one of the sequences SEQ ID NOs: 1-9 and 56;
(iii) the nucleotide sequence comprises the reverse complement of a sequence that is capable of hybridizing to the nucleotide sequence as set forth in (i) or (ii) under high stringency hybridization conditions or very high stringency hybridization conditions; and
(iv) the nucleotide sequence comprises a sequence having at least 90%, optionally at least 95%, preferably at least 97%, more preferably at least 98%, and most preferably at least 99% sequence identity to the nucleotide sequence as set forth in (i) or (ii).

In some embodiments, the target recognition site has a length of 6 nucleotides.

In some preferred embodiments, the target recognition site is programmable to hybridize to a different target sequence.

In some embodiments, the target nucleic acid is DNA or RNA.

In some embodiments, the major cleavage site of the DEAR nucleic acid manipulation system is 0-1 nt downstream of the 3' end of the target sequence in the target nucleic acid.

A second aspect of the present disclosure provides an isolated polynucleotide, wherein the polynucleotide comprises a nucleotide sequence encoding the DEAR nucleic acid manipulation system according to the first aspect of the present disclosure.

A third aspect of the present disclosure provides a nucleic acid construct, wherein the nucleic acid construct comprises the isolated polynucleotide according to the second aspect of the present disclosure.

A fourth aspect of the present disclosure provides a vector, wherein the vector comprises the isolated polynucleotide according to the second aspect of the present disclosure or the nucleic acid construct according to the third aspect of the present disclosure.

A fifth aspect of the present disclosure provides a cell, wherein the cell comprises the DEAR nucleic acid manipulation system according to the first aspect of the present disclosure, the isolated polynucleotide according to the second aspect of the present disclosure, the nucleic acid construct according to the third aspect of the present disclosure, or the vector according to the fourth aspect of the present disclosure.

A sixth aspect of the present disclosure provides a reagent or kit, wherein the reagent or kit comprises the DEAR nucleic acid manipulation system according to the first aspect of the present disclosure, the isolated polynucleotide according to the second aspect of the present disclosure, the nucleic acid construct according to the third aspect of the present disclosure, the vector according to the fourth aspect of the present disclosure, or the cell according to the fifth aspect of the present disclosure.

A seventh aspect of the present disclosure provides a pharmaceutical composition, wherein the pharmaceutical composition comprises the DEAR nucleic acid manipulation system according to the first aspect of the present disclosure, the isolated polynucleotide according to the second aspect of the present disclosure, the nucleic acid construct according to the third aspect of the present disclosure, the vector according to the fourth aspect of the present disclosure, or the cell according to the fifth aspect of the present disclosure; and optionally a pharmaceutically acceptable carrier.

An eighth aspect of the present disclosure provides a method for modifying a target nucleic acid, comprising the step of contacting the target nucleic acid with the DEAR nucleic acid manipulation system according to the first aspect of the present disclosure, the isolated polynucleotide according to the second aspect of the present disclosure, the nucleic acid construct according to the third aspect of the present disclosure, the vector according to the fourth aspect of the present disclosure, the cell according to the fifth aspect of the present disclosure, or the reagent or kit according to the sixth aspect of the present disclosure.

Provided is use of the DEAR nucleic acid manipulation system according to the first aspect of the present disclosure, the isolated polynucleotide according to the second aspect of the present disclosure, the nucleic acid construct according to the third aspect of the present disclosure, the vector according to the fourth aspect of the present disclosure, or the cell according to the fifth aspect of the present disclosure in modifying a target nucleic acid or in preparing a reagent or kit for modifying a target nucleic acid.

### Effects of the Invention

The RNA ribozyme-based DEAR nucleic acid manipulation system provided by the present disclosure is based on an RNA molecule derived from a bacterial Group IIC intron, avoiding the problems with the CRISPR-Cas system, such as the large size of protein molecules affecting transfection efficiency and the potential immunogenicity caused by Cas proteins. The RNA ribozyme-based DEAR nucleic acid manipulation system provided by the present disclosure can achieve the cleavage of DNA and RNA, and it also has the ability to cleave DNA in *E. coli* and mammalian eukaryotic cells.

### BRIEF DESCRIPTION OF THE DRAWINGS

FIGS. 1A-1J: Illustration of the secondary structures of DEAR1-DEAR10. FIGS. 1A-1J illustrate the secondary structure prediction results for DEAR1-DEAR10, respectively. The prediction is carried out using RNAfold. Domains I-VI and TRS have all been marked in the figure.
FIGS. 1K-1M: Primary sequence and secondary structure characteristics of Group IIC introns. Group IIC intron RNAs in the database (http://webapps2.ucalgary.ca/~groupii/) are modeled by the LocARNA software. Domains I-VI have been marked in the figure, corresponding to the primary sequence and secondary structure characteristics of Domain I, three primary sequence and secondary structure characteristics of Domains II-III (Models 1-3), and the primary sequence and secondary structure characteristics of Domains V-VI in the Group IIC introns, respectively.
FIG. 2A: Identification of the masses of RNA ribozyme molecules DEAR1-DEAR9.
FIG. 2B: Identification of the mass of RNA ribozyme molecule DEAR10.
FIG. 3: Verification of the RNA cleavage activities of RNA ribozyme molecules DEAR1-DEAR9.
FIG. 4: Verification of the cleavage activities of RNA ribozyme molecules DEAR1-DEAR6 on unpaired RNA substrates.
FIG. 5: Verification of the ssDNA cleavage activities of RNA ribozyme molecules DEAR1-DEAR6.
FIG. 6: Comparison of the cleavage of paired and unpaired DNA substrates by RNA ribozyme molecules DEAR1-DEAR6.
FIG. 7: Verification of the ssDNA cleavage sites of RNA ribozyme molecules DEAR1-DEAR6.
FIG. 8: Results of reaction condition optimization for RNA ribozyme molecule DEAR1.
FIG. 9: Efficiency curves of reaction condition optimization for RNA ribozyme molecule DEAR1.
FIG. 10: Comparison of the DNA cleavage activities of DEAR1 and RNA-guided protein nucleases.
FIG. 11: Verification of the plasmid cleavage activity of RNA ribozyme molecule DEAR1.
FIG. 12: Verification of the plasmid cleavage activities of RNA ribozyme molecules DEAR1-DEAR3 in *E. coli.*
FIG. 13: Further verification of the plasmid cleavage activity of RNA ribozyme molecule DEAR1 in *E. coli.*
FIG. 14: Verification of the plasmid cleavage activities of RNA ribozyme molecules DEAR4-DEAR9 in bacteria.
FIG. 15: Verification of the ssDNA cleavage activities of RNA ribozyme molecules DEAR1-DEAR6 with a reprogrammed TRS region.
FIG. 16: Schematic diagrams of the survival of the cells that are stably transfected with the DEAR1 stable transfection plasmid and the DEAR-NT stable transfection plasmid in Example 9.
FIGS. 17A-17B show schematic diagrams of the analysis of the sequencing results of Example 9.
FIG. 18: Verification of the RNA cleavage activity of DEAR10.
FIG. 19: Verification of the ssDNA cleavage activity of DEAR10.
FIG. 20: Comparison of the cleavage of paired and unpaired DNA substrates by DEAR10.
FIG. 21: Verification of the ssDNA cleavage activity of DEAR10 with a reprogrammed TRS region.
FIG. 22: Verification of the plasmid cleavage activities of DEAR1-DEAR6 and DEAR10.
FIG. 23: Toxicity test of DEAR in *E. coli.*
FIG. 24: Verification of the cleavage activity of DEAR on genomic DNA of mammalian cells, in which FIG. 24A shows a schematic diagram of a verification system for verifying the cleavage activity of DEAR on the mammalian cell genome; and FIG. 24B shows the survival of the DEAR-edited mammalian cells under resistance screening.
FIGS. 25A-25C: Detection of the editing pattern of DEAR on mammalian cells, in which FIG. 25A shows detection of the editing pattern of DEAR1 at three target sites; FIG. 25B shows detection of the editing pattern of DEAR1 over the entire target sequence; and FIG. 25C shows detection of the editing of DEAR1 on sequences upstream and downstream of the target site.

### DETAILED DESCRIPTION OF EMBODIMENTS

In order for the present disclosure to be more readily understood, certain technical and scientific terms are specifically defined below. Unless otherwise expressly defined herein, all other technical and scientific terms used herein have the meaning commonly understood by those of ordinary skill in the art to which the present disclosure belongs.

In this specification, the numerical range expressed as "numerical value A-numerical value B" refers to the range containing the endpoint numerical values A and B.

In this specification, the use of "substantially" or "essentially" means that the standard deviation from the theoretical model or theoretical data is within the range of 5%, preferably 3%, and more preferably 1%.

In this specification, the meaning expressed by "may" includes both the situation of carrying out a certain treatment and the situation of not carrying out a certain treatment.

In this specification, "optional" or "optionally" means that the subsequently described event or circumstance may or may not occur, and that the description includes instances where the event occurs and instances where it does not.

In this specification, reference to "some specific/preferred embodiments", "other specific/preferred embodiments", "embodiments", etc. means that the specific elements (e.g., features, structures, natures, and/or properties) described in connection with the embodiment are included in at least one of the embodiments described here, and may or may not be present in other embodiments. In addition, it is understood that the elements may be combined in various embodiments in any suitable manner.

In the present disclosure, the terms "comprising" and "having" and any variations thereof are intended to cover non-exclusive inclusion. For example, a process, method, device, product, or apparatus that includes a series of steps is not limited to the listed steps or modules, but may also optionally include steps not listed, or other steps inherent to such process, method, product, or apparatus.

In the present disclosure, reference to "a plurality of" means two or more. "And/or" describes an association relationship between associated objects, and represents that three relationships may exist. For example, A and/or B may represent the following three cases: only A exists, both A and B exist, and only B exists. The character "/" generally represents an "or" relationship between the associated objects.

In this specification, the terms "polynucleotide" and "nucleic acid" as used interchangeably refer to a polymeric form of nucleotides (ribonucleotides or deoxyribonucleotides) of any length. Thus, this term includes, but is not limited to, single-, double-, or multi-stranded DNA or RNA, genomic DNA, cDNA, DNA-RNA hybrids, or a polymer comprising purine bases and pyrimidine bases, or other natural, chemically or biochemically modified, non-natural, or derivatized nucleotide bases.

In this technical field, "G", "C", "A", "T", and "U" generally represent the bases guanine, cytosine, adenine, thymine, and uracil, respectively. However, it is also commonly known in this field that each of "G", "C", "A", "T", and "U" generally also represents a nucleotide containing guanine, cytosine, adenine, thymine, and uracil as the base, respectively, which is a common way of representing deoxyribonucleic acid sequences and/or ribonucleic acid sequences. Therefore, in the context of the present disclosure, the meanings represented by "G", "C", "A", "T", and "U" include the various possible situations described above. However, it is understood that the term "ribonucleotide" or "nucleotide" may also refer to a modified nucleotide or an alternative replacement moiety. One skilled in the art will appreciate that guanine, cytosine, adenine, and uracil can be replaced by other moieties without substantially altering the base-pairing properties of an oligonucleotide (including a nucleotide having such a replacement moiety).

In this specification, the term "nucleic acid manipulation" includes binding, nicking in one strand, or cleaving (i.e., cutting) both strands of a nucleic acid, or include modifying or editing nucleic acids. Nucleic acid manipulation can silence, activate, or regulate (increase or decrease) the expression of the RNA or polypeptide encoded by the nucleic acid.

In this specification, "hybridizable" or "complementary" or "substantially complementary" means that a nucleic acid (e.g., RNA or DNA) comprises a nucleotide sequence that enables the nucleic acid to non-covalently bind (i.e., form Watson-Crick base pairs and/or G/U base pairs), "anneal", or "hybridize" to another nucleic acid in a sequence-specific, antiparallel manner (i.e., the nucleic acid specifically binds to the complementary nucleic acid) under appropriate *in vitro* and/or *in vivo* temperature and solution ionic strength conditions. Standard Watson-Crick base pairing includes: adenine (A) pairing with thymidine (T), adenine (A) pairing with uracil (U), and guanine (G) pairing with cytosine (C). In addition, for hybridization between two RNA molecules (e.g., dsRNA), and for hybridization of a DNA molecule to an RNA molecule (e.g., when a DNA or RNA target nucleic acid base pairs with the target recognition site of the DEAR nucleic acid manipulation system), guanine (G) can also pair with uracil (U). For example, in the case of base-pairing of a tRNA anticodon with a codon in an mRNA, G/U base-pairing is responsible, at least in part, for the degeneracy of the genetic code.

Hybridization and washing conditions are well known, and are illustrated in Sambrook, J., Fritsch, E.F., and Maniatis, T., Molecular Cloning: A Laboratory Manual, 2nd ed., Cold Spring Harbor Laboratory Press, Cold Spring Harbor (1989), in particular Chapter 11 and Table 11.1 in the reference; and Sambrook, J. and Russell, W., Molecular Cloning: A Laboratory Manual, 3rd ed., Cold Spring Harbor Laboratory Press, Cold Spring Harbor (2001). The "stringency" of hybridization is determined by the temperature and ionic strength conditions.

In the present disclosure, "moderate stringency conditions", "moderate-high stringency conditions", "high stringency conditions", or "very high stringency conditions" describe the conditions for nucleic acid hybridization and washing. For guidance on performing hybridization reactions, see Current Protocols in Molecular Biology, John Wiley & Sons, N.Y. (1989), 6.3.1-6.3.6, which is incorporated herein by reference. Both aqueous and non-aqueous methods are described in this reference, and either can be used. For example, specific hybridization conditions are as follows: (1) low stringency hybridization conditions involve hybridization in 6× sodium chloride/sodium citrate (SSC) at about 45°C, followed by two washes at a temperature of at least 50°C in 0.2× SSC and 0.1% SDS (for low stringency conditions, the washing temperature can be raised to 55°C); (2) moderate stringency hybridization conditions involve hybridization in 6× SSC at about 45°C, followed by one or more washes at 60°C in 0.2× SSC and 0.1% SDS; (3) high stringency hybridization conditions involve hybridization in 6× SSC at about 45°C, followed by one or more washes at 65°C in 0.2× SSC and 0.1% SDS; and preferably (4) very high stringency hybridization conditions involve hybridization in 0.5 M sodium phosphate and 7% SDS at 65°C, followed by one or more washes at 65°C in 0.2× SSC and 1% SDS.

Hybridization requires that two nucleic acids contain complementary sequences, but base mismatches are possible. The conditions suitable for hybridization between two nucleic acids depend on the lengths and degree of complementarity of the nucleic acids, which are well-known variables in the art.

In the present disclosure, a DNA sequence that "encodes" a specific RNA is a DNA nucleotide sequence that is transcribed into the RNA. A DNA polynucleotide can encode an RNA (mRNA) that is translated into a protein (such that both DNA and mRNA encode a protein), or an RNA that is not translated into a protein (e.g., tRNA, rRNA, microRNA (miRNA), "non-coding" RNA (ncRNA), the DEAR nucleic acid manipulation system provided by the present disclosure, etc.).

In the present disclosure, the term "naturally occurring" or "unmodified" or "wild-type" as applied to a nucleic acid, polypeptide, cell, or organism refers to a nucleic acid, polypeptide, cell, or organism present in nature. For example, a polypeptide or polynucleotide sequence that exists in an organism and can be isolated from a source in nature is naturally occurring.

In the present disclosure, "recombinant" means that a specific nucleic acid (DNA or RNA) is the product of various combinations of cloning, restriction, polymerase chain reaction (PCR), and/or ligation steps, which generate constructs having structural coding sequences or non-coding sequences that are distinguishable from those in endogenous nucleic acids present in natural systems. A DNA sequence encoding a polypeptide can be assembled from cDNA fragments or a series of synthetic oligonucleotides to provide a synthetic nucleic acid capable of being expressed by recombinant transcription units contained in cells or cell-free transcription and translation systems. Genomic DNA containing related sequences can also be used in the formation of recombinant genes or transcription units. Sequences of untranslated DNA can be present at the 5' or 3' end of the open reading frame, where such sequences do not interfere with the manipulation or expression of the coding region, and can actually function by various mechanisms to regulate the production of the desired product (see "DNA regulatory sequence"). Alternatively, DNA sequences encoding non-translated RNA (e.g., the DEAR nucleic acid manipulation system provided by the present disclosure) can also be considered recombinant. Thus, for example, the term "recombinant" nucleic acid refers to a non-naturally occurring polynucleotide or nucleic acid, e.g., a polynucleotide or nucleic acid made by human intervention from an artificial combination of two otherwise separate segments of a sequence. Such artificial combinations are often accomplished by means of chemical synthesis or by manual manipulation of isolated segments of nucleic acids (e.g., by genetic engineering techniques). This procedure is typically performed to replace codons with those encoding the same amino acids, conserved amino acids, or non-conserved amino acids. Alternatively, this procedure is performed to link nucleic acid segments with the desired function together to produce the desired combination of functions. Such artificial combinations are often accomplished by means of chemical synthesis or by manual manipulation of isolated segments of nucleic acids (e.g., by genetic engineering techniques).

As used in the present disclosure, the term "isolated" means a substance in a form or environment that does not exist in nature. Non-limiting examples of isolated substances include (1) any non-naturally occurring substance; (2) any substance including, but not limited to, any enzyme, mutant, nucleic acid, protein, peptide, or cofactor, which has been at least partially removed from one or more or all of the naturally associated components with which it is inherently related; (3) any substance modified by an artificial means relative to a naturally found substance; or (4) any substance modified by increasing the amount of the substance relative to other components with which it is naturally associated (e.g., recombinant production in a host cell; multiple copies of a gene encoding the substance; and the use of a stronger promoter than that naturally associated with the gene encoding the substance).

As used in the present disclosure, the term "nucleic acid construct" comprises a polynucleotide encoding a polypeptide or domain or module operably linked to a suitable regulatory sequence that is necessary for expression of the polynucleotide in a selected cell or strain. In the present disclosure, transcriptional regulatory elements include promoters, and on this basis may further include elements such as enhancers, silencers, and insulators.

The term "vector" refers to a genetic element, such as a plasmid, cosmid, bacmid, phage, or virus, to which another genetic sequence or element (DNA or RNA) can be attached. The vector can be a replicon, resulting in the replication of the attached sequence or element. An "expression vector" is a vector that facilitates expression of a nucleic acid or a nucleic acid sequence encoding a polypeptide in a host cell or organism.

In the present disclosure, the term "recombinant expression vector" or "DNA construct" is used interchangeably herein to refer to a DNA molecule comprising a vector and an insert. Recombinant expression vectors are typically produced for the purpose of expressing and/or propagating one or more inserts, or for the purpose of constructing other recombinant nucleotide sequences. The one or more inserts may or may not be operably linked to a promoter sequence, and may or may not be operably linked to a DNA regulatory sequence.

As used herein, the term "operably linked" refers to a nucleic acid sequence that is placed into a functional relationship with another nucleic acid sequence. Examples of nucleic acid sequences that may be operably linked include, but are not limited to, promoters, transcription terminators, enhancers or activators, and heterologous genes, which, when transcribed and, if appropriate, translated, produce functional products such as proteins, ribozymes, or RNA molecules.

The term "derived from" as used herein refers to the origin or source, and may include naturally occurring, recombinant, unpurified, or purified molecules. A nucleic acid derived from the original nucleic acid can partially or completely comprise the parent nucleic acid, and can be a fragment or variant of the original nucleic acid.

In the present disclosure, the term "ribozyme" refers to an RNA molecule capable of catalyzing a specific biochemical reaction. Common examples of such reactions include cleavage or ligation of RNA and DNA, modifications, etc.

In the present disclosure, a "target nucleic acid" is a polynucleotide (e.g., DNA such as genomic DNA, RNA, etc.) that comprises a site ("target site" or "target sequence") targeted by the DEAR nucleic acid manipulation system provided by the present disclosure. The target sequence is a sequence to which the target recognition site of the DEAR nucleic acid manipulation system will hybridize. For example, the target site (or target sequence) within the target nucleic acid, 5'-UGUCUU-3' or 5'-TGTCTT-3', is targeted by (or bound by, hybridized or complementary to) the sequence 5'-AAGACA-3'. Suitable hybridization conditions include physiological conditions normally present in cells.

In the present disclosure, "cleavage" means the cleavage of the covalent backbone of a target nucleic acid molecule (e.g., RNA or DNA). Both single-strand and double-strand cleavage are possible, and double-strand cleavage can occur as a result of two distinct single-strand cleavage events. "Major cleavage site" refers to the DNA/RNA cleavage site corresponding to the cleavage product with a distinct band. "Minor cleavage site" refers to the DNA/RNA cleavage site corresponding to the cleavage product with a non-distinct band.

In the present disclosure, "stem-loop", also known as "hairpin", "hairpin loop", "stem-loop structure", or "stem-loop/hairpin structure", refers to a secondary structure formed by a single-stranded oligonucleotide when complementary bases in the first part of a linear chain hybridize to bases in the second part of the same chain.

The technical solutions of the present disclosure are specifically described below.

In the present disclosure, an RNA ribozyme-based DEAR nucleic acid manipulation system has been constructed based on a bacterial Group II intron element. Group II introns consist of two parts: an RNA ribozyme and an intron-encoded protein (IEP), in which the RNA ribozyme can catalyze the self-splicing and maturation of the primary transcript, while the protein IEP plays an auxiliary role. The RNA ribozyme portion comprises six domains, I-VI. Domain I, the largest among all domains, plays an important stabilizing role in the formation of the overall intron structure, and contains an exon binding site (EBS) for binding exons. Domains II and III also participate in the formation of the ribozyme structure. Domain IV contains an open reading frame (ORF), which encodes the protein IEP. Domain V serves as the catalytic center of the RNA ribozyme, while Domain VI performs an auxiliary catalytic function. Based on the RNA primary sequence and secondary structure characteristics, Group II introns can be classified into classes A, B, and C, among which class C is considered the more ancient class of introns (D. M. Simon et al., Group II introns in eubacteria and archaea: ORF-less introns and new varieties. RNA 14, 1704-1713 (2008); A. M. Lambowitz, S. Zimmerly, Mobile group II introns. Annu Rev Genet 38, 1-35 (2004); and J. S. Rest, D. P. Mindell, Retroids in archaea: phylogeny and lateral origins. Mol Biol Evol 20, 1134-1142 (2003).).

In the present disclosure, it has been found that by using an EBS of a Group IIC intron and its adjacent sequences as the substrate recognition element (referred to in the present disclosure as target recognition site (TRS)) of the target nucleic acid for a ribozyme, the programmability of TRS has been found and demonstrated, and the target nucleic acid (RNA or DNA) is hydrolytically cleaved by Domain V of the RNA intron ribozyme. Therefore, in the present disclosure, the system constructed based on a bacteria-derived Group IIC intron in which an open reading frame encoding an intron-encoded protein is present or absent in Domain IV, and having programmable nucleic acid recognition and cleavage capabilities, is referred to as an RNA ribozyme-based DEAR (Dr) nucleic acid manipulation system, or an RNA ribozyme-based HYER (Hr) nucleic acid manipulation system.

### <DEAR nucleic acid manipulation system>

In some embodiments of the present disclosure, provided is an RNA ribozyme-based DEAR nucleic acid manipulation system, comprising an (isolated) RNA molecule derived from a bacterial Group IIC intron, wherein the RNA molecule comprises a target recognition site that hybridizes to a target sequence in a target nucleic acid.

In some embodiments of the present disclosure, the DEAR nucleic acid manipulation system comprises at least one of Domain I, Domain II, Domain III, Domain IV, Domain V, and Domain VI.

In some preferred embodiments, the DEAR nucleic acid manipulation system comprises at least Domain I, Domain II, Domain III, and Domain V.

In some specific embodiments of the present disclosure, the DEAR nucleic acid manipulation system contains 6 domains (i.e., Domain I, Domain II, Domain III, Domain IV, Domain V, and Domain VI; and the 6 domains may also be represented as Domains I-VI, or simply D1-D6) in the range of 100-5660 nt, and preferably 124-3897 nt, in length.

In some specific embodiments of the present disclosure, Domain I comprises 2-6 stem-loop/hairpin structures in the range of 50-400 nt in length, and a TRS sequence responsible for substrate recognition, and preferably 3-5 stem-loop/hairpin structures in the range of 65-384 nt in length; Domain II comprises 1-4 stem-loop/hairpin structures in the range of 10-300 nt in length, and preferably 1-3 stem-loop/hairpin structures in the range of 10-218 nt in length; Domain III comprises 1-3 stem-loop/hairpin structures in the range of 10-200 nt in length, and preferably 1-2 stem-loop/hairpin structures in the range of 10-140 nt in length; Domain IV comprises 0-4 stem-loop/hairpin structures in the range of 0-4500 nt in length, and a 0-4000-nt region as an open reading frame encoding an IEP, and preferably 0-4 stem-loop/hairpin structures in the range of 0-3000 nt in length; Domain V comprises 1 stem-loop/hairpin structure in the range of 20-60 nt in length, and preferably 1 stem-loop/hairpin structure in the range of 29-43 nt in length, which comprises a catalytic core; and Domain VI comprises 1 stem-loop/hairpin structure in the range of 10-200 nt in length, and preferably 1 stem-loop/hairpin structure in the range of 10-112 nt in length.

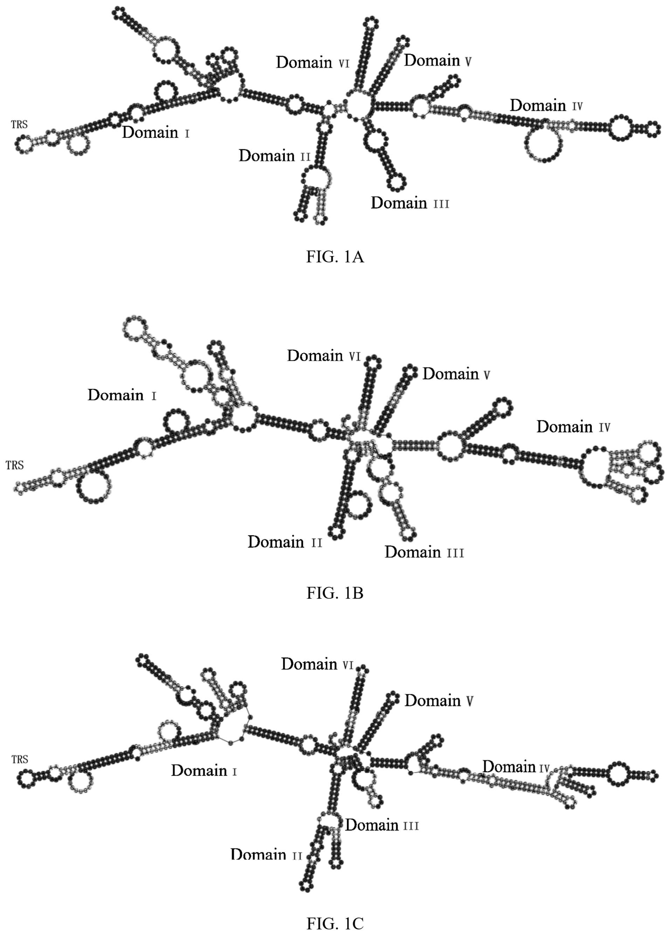

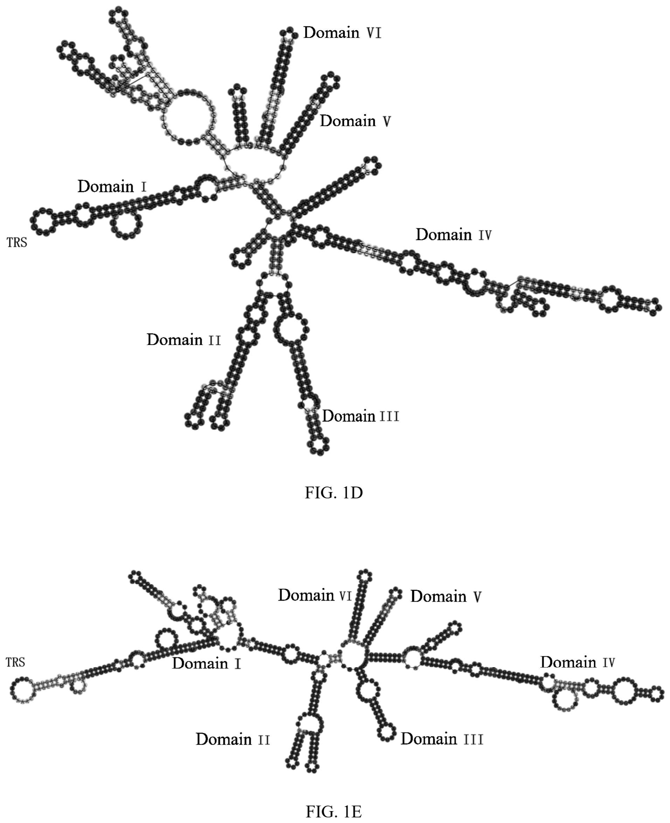

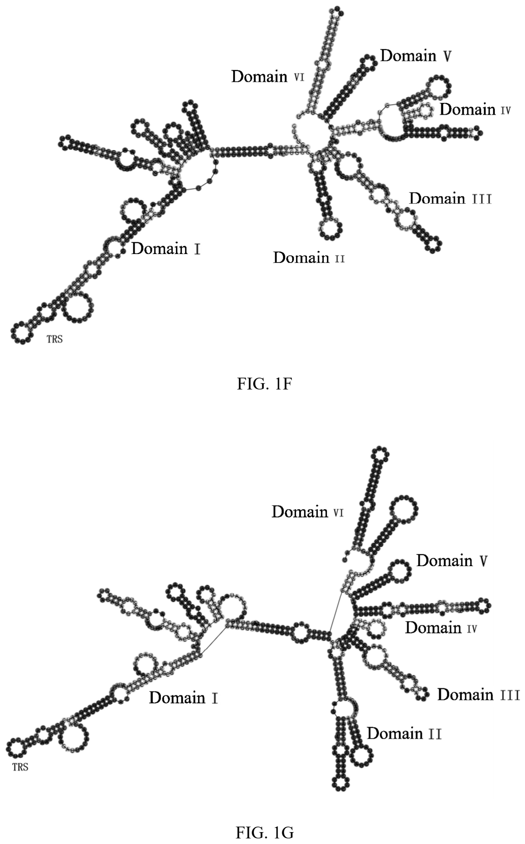

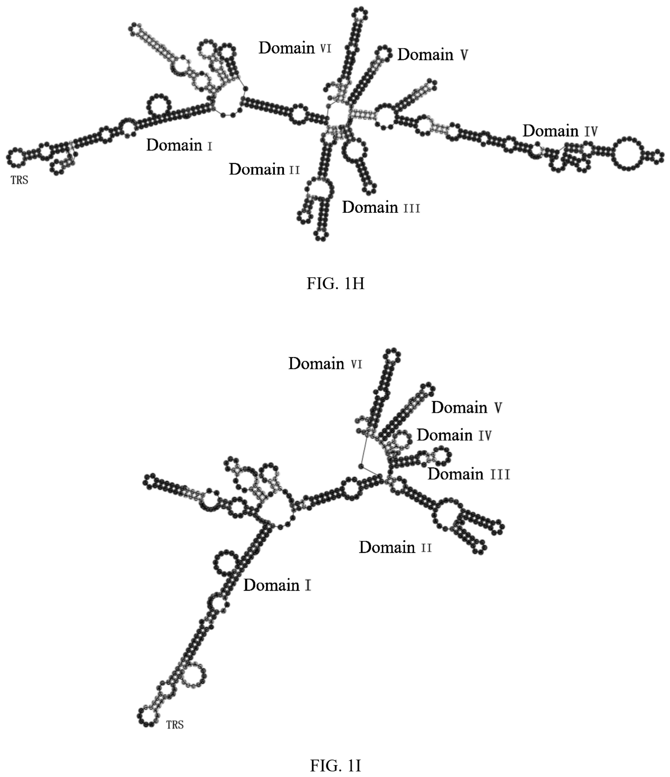

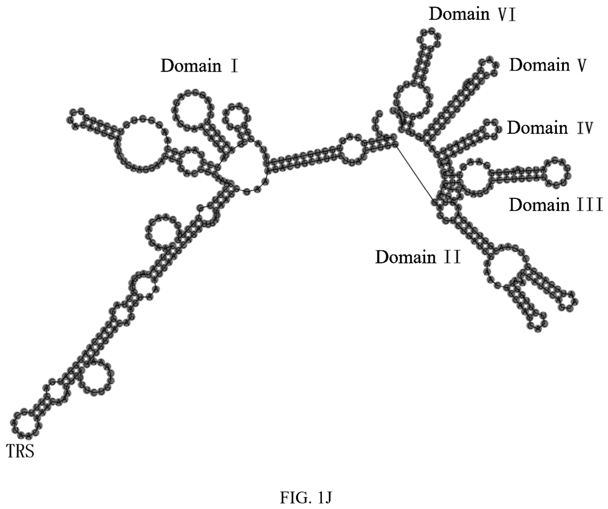

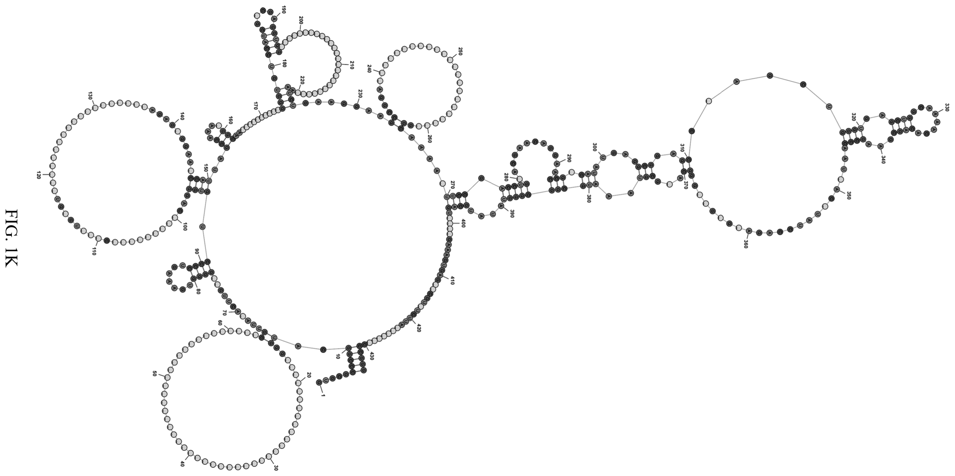

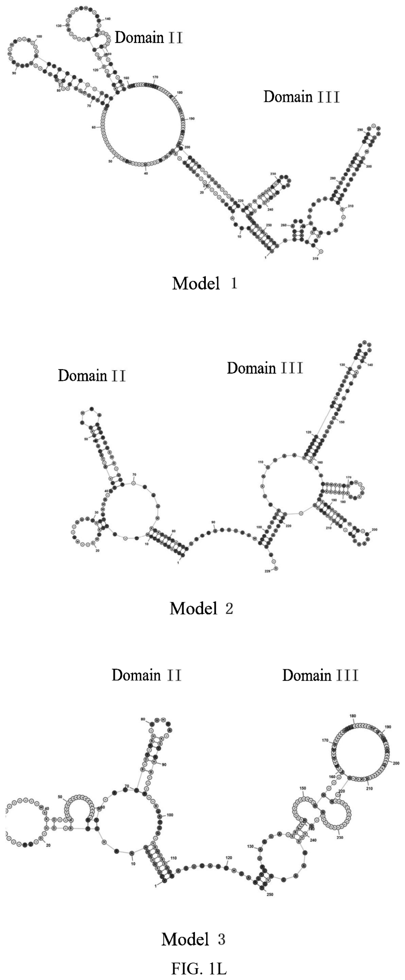

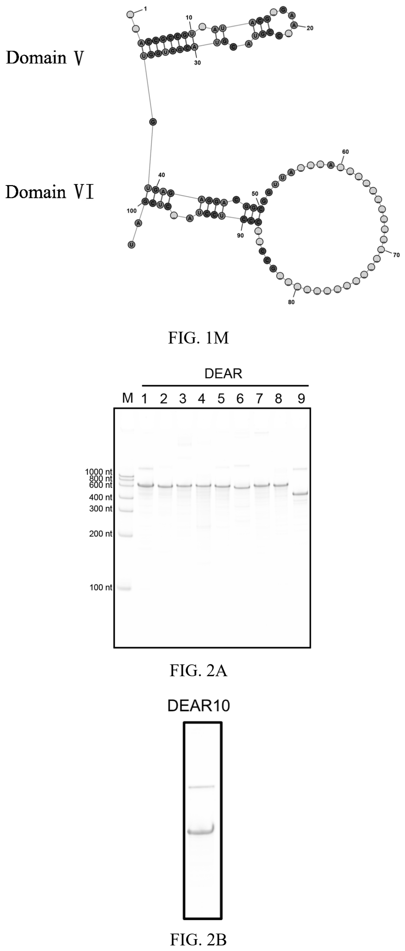

The primary sequence and secondary structure characteristics of the RNA molecules derived from the Group IIC introns are shown in FIGS. 1K-1M.

In some embodiments of the present disclosure, the Group IIC intron is a Group IIC intron in which an open reading frame encoding an IEP is absent in Domain IV.

In other embodiments of the present disclosure, the Group IIC intron is a Group IIC intron in which an open reading frame encoding an IEP is present in Domain IV. In some more specific embodiments, in the Group IIC intron in which an open reading frame encoding an IEP is present, the open reading frame encoding an IEP is deleted.

The DEAR nucleic acid manipulation system provided by the present disclosure functions as an endonuclease, which catalyzes nucleic acid cleavage at a specific sequence in the targeted target nucleic acid (e.g., DNA or RNA). As will be detailed hereafter, sequence specificity is provided by the target recognition site in the DEAR nucleic acid manipulation system, which hybridizes to the target sequence in the target nucleic acid. Thus, the DEAR nucleic acid manipulation system binds to the target nucleic acid by hybridizing the target recognition site to the target sequence in the target nucleic acid. In other words, the location at which specific binding (and/or cleavage) of the target nucleic acid occurs is determined by the base-pairing complementarity of the target recognition site with the target nucleic acid.

In some specific embodiments, the major cleavage site of the DEAR nucleic acid manipulation system is 0-1 nt downstream of the 3' end of the target sequence in the target nucleic acid, that is, the major cleavage site is located 0-1 nt downstream of the 3' end of the region paired with the target recognition site on the target nucleic acid.

In some embodiments of the present disclosure, the nucleotide sequence of the RNA molecule of the DEAR nucleic acid manipulation system is selected from any one of:
(i) the nucleotide sequence comprises a nucleotide sequence as set forth in any one of the sequences SEQ ID NOs: 1-9 and 56;
(ii) the nucleotide sequence comprises a nucleotide sequence that is the reverse complement of a sequence as set forth in any one of the sequences SEQ ID NOs: 1-9 and 56;
(iii) the nucleotide sequence comprises the reverse complement of a sequence that is capable of hybridizing to the nucleotide sequence as set forth in (i) or (ii) under high stringency hybridization conditions or very high stringency hybridization conditions; and
(iv) the nucleotide sequence comprises a sequence having at least 90%, optionally at least 95%, preferably at least 97%, more preferably at least 98%, and most preferably at least 99% sequence identity to the nucleotide sequence as set forth in (i) or (ii).

SEQ ID NOs: 1-9 and 56 are shown below:

| SEQ ID NO: | RNA sequence (5'-3') |
|---|---|
| 1 | GUGCGCUCGGCAUGGGUGCAAUCUCUAGGGUGAAAGUCCCGAACUGCGAAGGCAGAAGUA GCAGUUAGCUUAACGCAAGGGUGUCCGUGGUGACGCGGAAUCUGAAGGAAGCGGGCGGCA AACUUCCGGUCUGAGGAACACGAACUUCAUAUAAGGCUAGGUAUCAUUGGAUGAGUUUGC NNNNNNAAACAAAGUCCUUUCUGCCGAAGGUGAUACAGAGUAAAUGAAGCAGAUAGAUGG AAGGAAAGAUUGUACUCUUACCCGAGGAGGUCUGAUGGAUACGUGAAGUGCGCUUCAUAA CCUACUUAGUGAUAAGUAACUGAACCAUCAGAAGUCAGCAGAGGUCAUAGUACGAAUCGG UCUAGAACGAUUCGGAAGGACUGAACAAUCAAGAGAAAAUAGCCCUUGGCAUUCAGUACG UCAUGAUGAACACAGAAAACAUGGUACCUCCCAAGAGAAAGGAAACGGUGAAUCCCGUGG GAAUCUUUUGGAGGGUGGAGUGACGACUGGCAUAAGAAGAUCAGCUAUUUACGGAAGGAA GCUUGCGUCAUUAUCUUGAUUGAACCGCCGUAUACGGAACCGUACGUACGGUGGUGUGAG AGGACGGAGGUUAAUCACCUCCUCCUACUCGAU |
| 2 | GUACGACGGGCAUGUCGUACAUCUGAGGUGCAAGUCCUCUGUGGACACCUGCUACCGGUG AACCCAAUAGCGACUCCCAAGCCUGACUAUCGCGAGGUAACGGGGAGAGGAAGGGAUAGC GAAAUCGUGGCUCUACGAACAGAAACGUGAUAUUAAGGCGUAUAUAGUGGAUGAGGGGGC NNNNNNCUCUAAAGUCCAAAAAGGUAGUCGUAAUCUAUGUGCGUAAAUCACGAGAGUAAA CGAGGAAAGAUGUACGACUUAUCCCGUGAGGUCUCAUGAGCGUCAAGUAGACGUAGUAAU AACGAAUCAUGAGAAGUCAGCCGAGGUCAUAGUAGUGAUGAAGUUUCUGUAAUGGAAACG GAGCGAAGGACCGAACGAUUAAAUGAUACCCUAUCUAACUGAUUUUGUCUGCCCUAACAC CUGGUCUGAAAUGACGGUACUCGCAGAACGUAUGCUGAAAGAGGCUGCUCACAAAUAUAG GACGGCACAAGCGGAACGAAAUCAUAUAUAGGUAGGUGUGGGAACAAAAAAAGUGUUCUC GCCAAAGUUUAGUUGAACCGCCGUGUGCCGAACGGCACGCACGGUGGUGUGAGAGGGACU AUCAAUUAGUCCCUACUCGAU |
| 3 | GUAUGCCCGGCAUGGGCGAUAGCUUUAGGGUGAAAGUCCCGAACAGGGGUUGGCAACACC AACUGUUAGCCAAGAGCAAGGGUGUCCAUCGUGAGGUGGAAUCUGAAAGAAGCUGGAGGC AAAUUCCCGACCUGAGGUACACGAAUCACAUUCGAGGCUGUUAUAUUCGGGCAAGACUGC NNNNNNAGUCAAAGCCCUAUGUUGCCAAGGGAUAUAGCAGUAAAUGUGGCAGGUACAUGG GAUGAAAGUUAUCGCUCUUAUCCGGGGAGGUCUGUCGAUCCGGUAAUGGUAAGGAAGUCC UUAACAAUACCAACCCGCAUCGUGAGGUGUCGGCUGAGUCGGCAGAAGUCAGCAGAGGCC AUAGUACUCCUAACGAGGAAGGGCCGAAGGUCAGGGCAGACUAAGAACGGUGCGUUCGAG AGAAUUUGCUGAUAGCAGUUGUCUCUGAAACAGAGACCUAUCUAUAAGAGGGAGUGGUGA AGCCACAAUGGCUUAUAGAAGGGCUGAGAAUUCUCCGGCACAAUACCCGAAAAUCUUAAU CUUAAUCGCUGAGUAAUCAGUUAACCCUGGCCAACCGCCGGAUGCGGACCCGCAUGUCCGG UGGUGUGGGAGGGUCGGCGGAGAUUAUCUCCGCCCCCUAUCCCAAU |
| 4 | GUGCGCCCAGCAUGGGCGCAAUCUUGGAGGUGAAAGUCCUCCCGUAAGUUGAUCACAGCG AACGAAGCGAAGCGCAACUGCAUGAGGGCGACUGAGUGUGGGGAGGAAGCGUGGAGCGAA |
| | GCUGCGAGCCGAUGAACAAGAAUCGGAUAGAAGGCGGUGCCGACCAGGGCGAGCGGGCNN NNNNCCGCGAAGCUCUCGUGAUCAAGGGUCAGGCGGCGUAGAUCCGGCGGUUGUGCAGUG AAGGAUUGCGUUCUUACCUGGGGAGAUCUCGCUUUGUGCCUGAAAGGGCGACGGUGUAAG CCGGAGCGAGAAGUCAGCAGAGGUCGUAGUAGUUGGCGGCAUGGCCGGAGAGGCUGGAAG CUGUUGGCGAAGGACCAAAGGAGCAGAAGGGCAAGUCUGCCUGACCAUCCGAAAUGGAUA UGCUUCAGAUGCCCGGAUACGGGGCUCGUUACGAAAAGUGCAGCAGGUGAAGCCUGAAAU ACUUGUGACAGCGAAGAACCAGAUCGGCCGGGUUGGGGACAGGCGAGUCCAGGCGUCAUG UUCGGGUAUGGGCAUGACGACCUCAACCGUUUCAACCGCCCGGUGCGGACCCGCAUGCCGG GUGGUGUGGCAGGGGACCGGUCAGUUAUACUGACCGCCCCUAUGCCGAU |
| 5 | GUGCGCCCGGCAUGUACAUGAACUAUAGGGUGUAAGUCCCGAACCCCGAAGACAGAAGUA GAGGUUAGCCAAGAGCAAGGGUGUCCGUGGUGACGCGGAAUCUGAAGGAAGCUGGAGGCA AAACACCGGUCCGAGGAACACGAACCUCAUAUAAGGCUAGGUAUGAUUGAGUGAGUUUGC NNNNNNAAACAAAGCUCUUUCUGUCGAAGGUCAUAUCGAGUAAAUGAGGCGGAUAGAUGG UGUGAAAGUGCAUGUACUUACCCGGGGAGGUCUGGCGGAUAUGUGAAGUACUCUUCAUAA CCUACUUAGUGAUAAGUAGCUGAACCGUCAGAAGUCAGCAGAGGUCAUAGUAUUAGUUGG UCUAGAACAACUAAGAAGGACCGAACAAUUAAGAGAGAAUAGCCCUUGGUAUUCAGUGAG UCAUGAUGAACACAGAAAACGUAGUACCUCACUUGAGGGAGGAAGCGGUGAAUCCCGUGG GAGACCUCUUGGAGGGUGGAGUGACCACUGGCAUAAAGAGAACAGCUAUUCACGGAAGUU AUAAAGACUUGCGUCAAUUAUCUUAAUUGAACCGCCGUAUACGGAACCGUACGUACGGUG GUGUGAGAGGACGGGAGUUAAUCGCUCCCUCCUACUCGAU |
| 6 | GUGUGCCCAGUAUGGGCAUCUUUUGUUUUACCGAAAGGUAAAAUAUUAAUCUAGAGGGUG CAAGUCCCUUAUGGGCAGGGGUAACGCCUUGAACCAUUAGUAAGCCGCAAGGGUGGUGAU CGUGAGGUCACAUCUGAAGAAAGCGGGACUACAAAACCCGGUACUGACGAACAGGAACCG UAUACAGAGGCAUUUAUGCAUGGGUAAGCAACCNNNNNNUUGUAAAGCCCUAAGAAUACC AAAAGUGCAUUUAUGUAGAUACGGCAGGGAUCGGGGGAAAGAAGAUGCUCUUACCUGGGG AGGUCUCCAGUACCACGUGUUUGUACAGGAGAAGUCAGCAGAGGUCAUAGUACCUAAGGG AAACGAGUUGCAGCGAAACUGCAUAGGUCUCACAAUUAGGAAAGGACCGAACGUAAUCCU CUUCAAAAUUCGCAUAGGAACACAACUUGUGUAGCCUAUGCUUAAACUAGAAGAUAGUGC CAAGGGUGAAAAGAGCUUUGGUGUGAUGGUUUACGAACCGCCGUAUACGAGACCCGUACG UGCGGUGGUGUGAGGGGUGCACUUCGUCAUUUGAUGGCGGAGCCAUCCACUCGAU |
| 7 | GUGUGCCCUGAAUGGCAAAUAUAGUUCUUUCAAAUAGAAUAAUCCAGAGAGUGAAAGUCU CUUAUGCGCCUCUUAAAUUGAGGAAGCAUUAGCAAGUCGCAAGGUGGUUUGUCGUGAGAU AUGCACUGAAGUAAGCGAGACUGCAAAGGUCGGUACUGACGUACAGAAACUGUAUAUAGA GGCAUGAUGAAUCGGGUGAGGUAGCNNNNNNUACUAAAGCCCCAAGAUUACAAACCAUUC AUCAUGUAGAUGCAGCAGCGAUUGACCGAAGGAUAUUUGUCUUACCGGGGGAGGUCUGUU GAUAGGUUGACUAAGAUUGGCGCGACAAUCGAAGCAACAAUUCCCGAAAGCAAUAGGGAG UUUCAAUAGAAGUCAGCAGAGGUCAUAGUAGUUGGUAGUUACGAGCCGAAGAUAAAUCGG ACGGUCUCACAAGCCAGCGAAGGACUGAAUGUUAAGUCGUUUCUAAUGCAAUAAGGAGCU UUAAGCUUCGCGCUUAGCAGCCUUGUUAUAAAAGAAACAAAGUGCAAUUGGUGAAAAGAG CGAUUGUACUCAUGCUUAACAAACCGCCGUGUACGAGACCCAUACGCACGGUGGUGUGAG GGGCACUCCCCACCUGUUCAUUCAGGUGGGGCUGUCCACUCGAU |
| 8 | GUGCGCCCAGCAUGUGCAAUAGCUAAGCGGUGAAAGUCCGUUGUGGGCCAGGUAGCGGGA ACCACUAGUCGAAGGCAAGGGUGUCCAUCGUGAGGUGGAAUCUGAAGGAAGCCCAAGGCA AAGUCCCGGUCCGAUGAACAAGAACCAGAUAUAAGGCUAAGUGAAGUGGACGAGUUUGCN |
| | NNNNNAAACGAAGUCCAACAGCUACCCUGAACUUCACGGAGUAGAUCUGGCGGAUAGAUG GGAUGAAAGUUAUUGCACUUACCUGGGGAGAUCUCAAGAAUAUGCUAUUGAGGCAACCCA UACAGCAAUGUAUGGCUGAAUCUUGAGAAGUCAGCAGAAGUCAUAGUACUCAUUGAGAGA UGAGGAAGGACUGAACAACAGAAGGUUUCGAAUCUUGAAAGAUGCGAAGGGAUACGGAAA AGGCAUACAACUUCGACUAGAAGGUUUCCUACAUGAAGAUAAGGGAGAGCCCGAAAAUAA UGUACCAAGCGCCUAGUAUAACUUCUACAUCUGAAAGAGGGAGAAACGAUGUGAAGGGUC UUAUACAUCAAACCUGUUGAACCGCCGUAUACAGAAUGGUACGUACGGUGGUGCGGAAGG ACGGCAGAUAAAUUAAUUAUCUACCUCCUACUCGAU |
| 9 | GUGCGCCCGGCAUGCGUAUGAACUAUAGGGUGCAAGUCCCGAACCCCGAAGACAGAAGUA GAGGUUAGCCAAGAGCAAGGGUGUCCGUGGCGACGCGGAAUCUGAAGGAAGCUGGAGGCA AAACACCGGUCCGAGGAACACGAAUCUCAUAUAAGGCUAGGUAUGAUUGGAUGAGUUUGC NNNNNNAAACAAAGUCCUUUCUGUCGAAGGUCAUAUCGAGUAAAUGAGGCGGAUAGAUGG UGUGAAAGUGCAUACGCUUACCCGGGGAGGUCUGAUGGAAACGUGAAGUACCCUUCAUAA CCUACUUGGUGACAAGUAGCUGAACCAUCAGAAGUCAGCAGAGGUCAUAGUACGAAACUU UGCGUUAUUCAUCUUAAUUGAACCGCCGUAUACGGAACCGUACGUACGGUGGUGUGAGAG GUCGGGAGUUAAUCACUCCCUCCUACUCGAU |
| 56 | GUGUGCCCGGCAUGGGUGCAGUCUAUAGGGUGAGAGUCCCGAACUGUGAAGGCAGAAGUA ACAGUUAGCCUAACGCAAGGGUGUCCGUGGCGACAUGGAAUCUGAAGGAAGCGGACGGCA AACCUUCGGUCUGAGGAACACGAACUUCAUAUGAGGCUAGGUAUCAAUGGAUGAGUUUGC NNNNNNAAACAAAGUCCUUUCUGCCAAAGUUGGUACAGAGUAAAUGAAGCAGAUUGAUGA AGGGAAAGACUGCAUUCUUACCCGGGGAGGUCUGAUCGAAACGCCAAGCACUCUUGGUAA CCCAUUCAGCAAUGGAUGGCUGAACGGUCAGAAGUCAGCAGAAGUCAUAGUACCCUGCAU ACUCGAGAAUGUAAGGGGAAGGACGGAACAAUUAAGUUCGCUUAAUUGAACCGCCGUAUA CCGAACGGUACGUACGGUGGUGUGAGAGGACGGGGGUUAGUCGCUCCCUUCUACUCUAU |

in which "NNNNNN" is the target recognition site, where N is A, U, G, or C.

In some specific embodiments, the DEAR nucleic acid manipulation system comprises an RNA molecule, wherein the nucleotide sequence of the RNA molecule is a nucleotide sequence as set forth in any one of the sequences SEQ ID NOs: 1-9 and 56.

In some preferred embodiments, the DEAR nucleic acid manipulation system comprises an RNA molecule, wherein the nucleotide sequence of the RNA molecule is a nucleotide sequence as set forth in any one of the sequences SEQ ID NOs: 1-6 and 56.

In some further preferred embodiments, the DEAR nucleic acid manipulation system comprises an RNA molecule, wherein the nucleotide sequence of the RNA molecule is a nucleotide sequence as set forth in any sequence of SEQ ID NOs: 1-3, 5, and 56.

### (Target recognition site)

In some embodiments, the target recognition site of the DEAR nucleic acid manipulation system is a nucleotide sequence that is complementary to a sequence in a target nucleic acid (target sequence). In other words, the target recognition site of the DEAR nucleic acid manipulation system can interact with the target nucleic acid (e.g., DNA or RNA) in a sequence-specific manner via hybridization (i.e., base pairing). The target recognition site can be modified (e.g., by genetic engineering)/designed to hybridize to any desired target sequence within a target nucleic acid (e.g., a prokaryotic target nucleic acid, a eukaryotic target nucleic acid, or an isolated target nucleic acid).

In some embodiments, the target recognition site is programmable in that it can be designed or engineered to recognize and bind to a different target sequence.

In some embodiments, the percent complementarity between the target recognition site and the target sequence of the target nucleic acid is 60% or more (e.g., 65% or more, 70% or more, 75% or more, 80% or more, 85% or more, 90% or more, 95% or more, 97% or more, 98% or more, 99% or more, or 100%). In some embodiments, the percent complementarity between the target recognition site and the target sequence of the target nucleic acid is 80% or more (e.g., 85% or more, 90% or more, 95% or more, 97% or more, 98% or more, 99% or more, or 100%). In some embodiments, the percent complementarity between the target recognition site and the target sequence of the target nucleic acid is 90% or more (e.g., 95% or more, 97% or more, 98% or more, 99% or more, or 100%). In some embodiments, the percent complementarity between the target recognition site and the target sequence of the target nucleic acid is 100%.

In some embodiments, the target recognition site has a length of 6 nucleotides (nt). In some specific embodiments, the sequence of the target recognition site is N₁N₂N₃N₄N₅N₆; in which N₁-N₆ are A, G, C, or U, respectively.

In some embodiments, at least any four nucleotides in the target recognition site of the DEAR nucleic acid manipulation system are complementary to the target sequence of the target nucleic acid. In some preferred embodiments, at least any five nucleotides in the target recognition site of the DEAR nucleic acid manipulation system are complementary to the target sequence of the target nucleic acid. In some more preferred embodiments, 6 nucleotides in the target recognition site of the DEAR nucleic acid manipulation system are complementary to the target sequence of the target nucleic acid.

In some specific embodiments, the sequence of the target recognition site is selected from, but not limited to:
(a) AAGACA;
(b) UAGGCA;
(c) CAGACA;
(d) AAUGAA;
(e) AUAACA;
(f) ACAUCA;
(g) CACUCA; and
(h) AUUACA.

In some specific embodiments, the DEAR nucleic acid manipulation system comprises an RNA molecule, wherein the nucleotide sequence of the RNA molecule is a nucleotide sequence as set forth in any one of the sequences SEQ ID NOs: 10-18 and 57.

In some specific embodiments, the sequence of the target recognition site is
(i) CGAUAG.

### (Target nucleic acid)

In the present disclosure, the DEAR nucleic acid manipulation system can bind to and cleave a target nucleic acid. In the present disclosure, the target nucleic acid can be any nucleic acid (e.g., DNA or RNA), can be any type of nucleic acid (e.g., chromosomal (genomic) DNA, chromosome-derived, chromosomal DNA, plasmid, viral, extracellular, intracellular, mitochondrial, chloroplastic, linear, circular, etc.), and can be derived from any organism (e.g., provided that the DEAR nucleic acid manipulation system comprises a nucleotide sequence that hybridizes to a target sequence in the target nucleic acid, such that the target nucleic acid can be targeted).

In particular, in the present disclosure, the target nucleic acid may be DNA or RNA. In some exemplary embodiments, the target nucleic acid is selected from: mRNA, rRNA, tRNA, non-coding RNA (ncRNA), long non-coding RNA (lncRNA), and microRNA (miRNA). In some exemplary embodiments, the target nucleic acid is viral DNA or plasmid DNA. The target nucleic acid can be located anywhere, e.g., outside of a cell *in vitro,* inside a cell *in vitro,* inside a cell *in vivo,* or inside a cell *ex vivo.*

### <Biomaterials>

### (Isolated polynucleotide)

In some embodiments of the present disclosure, an isolated polynucleotide is provided, wherein the polynucleotide comprises a nucleotide sequence encoding the DEAR nucleic acid manipulation system of the present disclosure.

### (Nucleic acid construct)

In some embodiments of the present disclosure, a nucleic acid construct is provided, wherein the nucleic acid construct comprises the isolated polynucleotide of the present disclosure.

In some optional embodiments, the polynucleotide is operably linked to one or more regulatory sequences, which are nucleotide sequences comprising a promoter and/or a ribosome binding site, and direct the expression of the gene of the DEAR nucleic acid manipulation system in a host cell.

### (Vector)

In some embodiments of the present disclosure, a vector is provided, wherein the vector comprises the isolated polynucleotide of the present disclosure or the nucleic acid construct of the present disclosure.

In some specific embodiments, the vector is a recombinant expression vector.

Suitable recombinant expression vectors include viral expression vectors (e.g., viral vectors based on the following viruses, vaccinia virus, poliovirus, adenovirus, adeno-associated virus, SV40, herpes simplex virus, and human immunodeficiency virus, retroviral vectors (e.g., murine leukemia virus, spleen necrosis virus, and vectors derived from retroviruses such as Rous sarcoma virus and Harvey sarcoma virus), avian leukosis virus, lentivirus, human immunodeficiency virus, myeloproliferative sarcoma virus, and mammary tumor virus), etc.).

### (Cell)

In some embodiments of the present disclosure, the present disclosure provides a cell, comprising the DEAR nucleic acid manipulation system of the present disclosure, the isolated polynucleotide of the present disclosure, the nucleic acid construct of the present disclosure, or the vector of the present disclosure.

The cell can be any of a variety of cells, including, for example, *in vitro* cells, *in vivo* cells, *ex vivo* cells, primary cells, cancer cells, animal cells, plant cells, algae cells, fungal cells, etc.

In some embodiments, the cell is a recipient of the DEAR nucleic acid manipulation system, isolated polynucleotide, nucleic acid construct, or vector provided by the present disclosure, and may also be referred to as a "host cell" or "target cell". The host cell or target cell may be a recipient of the DEAR nucleic acid manipulation system, isolated polynucleotide, nucleic acid construct, or vector provided by the present disclosure.

In some specific embodiments, non-limiting examples of cells include: prokaryotic cells, eukaryotic cells, bacterial cells, archaeal cells, cells of single-celled eukaryotic organisms, protozoan cells, cells from plants, algae cells, fungal cells, animal cells, cells from invertebrates, cells from vertebrates, cells from mammals (e.g., ungulates, rodents, non-human primates, humans, felines, canines, etc.), etc. In some cases, the cell is a cell that is not derived from a natural organism (for example, the cell can be a synthetic cell; also known as artificial cell).

Depending on the host/vector system used, any of a number of suitable transcriptional and/or translational control elements may be used in the recombinant expression vector, including constitutive and inducible promoters, transcriptional enhancer elements, transcription terminators, etc.

Methods for introducing nucleic acids into host cells are known in the art, and any convenient method can be used to introduce a nucleic acid (e.g., the recombinant expression vector, isolated polynucleotide, nucleic acid construct, or DEAR nucleic acid manipulation system provided by the present disclosure) into the cells. Suitable methods include, for example, viral infection, transfection, lipofection, electroporation, calcium phosphate precipitation, polyethyleneimine (PEI)-mediated transfection, DEAE-dextran mediated transfection, liposome-mediated transfection, particle gun technology, direct micro-injection, nanoparticle-mediated nucleic acid delivery, etc.

### <Reagent, kit, and pharmaceutical composition>

In some embodiments of the present disclosure, the present disclosure provides a reagent or kit, comprising the DEAR nucleic acid manipulation system of the present disclosure, the isolated polynucleotide of the present disclosure, the nucleic acid construct of the present disclosure, the vector of the present disclosure, or the cell of the present disclosure.

In some embodiments of the present disclosure, the present disclosure provides a pharmaceutical composition, comprising the DEAR nucleic acid manipulation system of the present disclosure, the isolated polynucleotide of the present disclosure, the nucleic acid construct of the present disclosure, the vector of the present disclosure, or the cell of the present disclosure, and optionally a pharmaceutically acceptable carrier.

### <Method and use of modifying target nucleic acid>

The present disclosure provides a method for modifying a target nucleic acid, comprising the step of contacting the target nucleic acid with the DEAR nucleic acid manipulation system of the present disclosure, the isolated polynucleotide of the present disclosure, the nucleic acid construct of the present disclosure, the vector of the present disclosure, the cell of the present disclosure, or the reagent or kit of the present disclosure. In some embodiments, the contacting results in modification of the target nucleic acid by the DEAR nucleic acid manipulation system.

The present disclosure provides use of the DEAR nucleic acid manipulation system of the present disclosure, the isolated polynucleotide of the present disclosure, the nucleic acid construct of the present disclosure, the vector of the present disclosure, or the cell of the present disclosure in modifying a target nucleic acid or in preparing a reagent or kit for modifying a target nucleic acid.

In some specific embodiments, the modification is cleavage of the target nucleic acid. In some specific embodiments, the target nucleic acid is selected from: DNA, RNA, genomic DNA, and extrachromosomal DNA.

In some specific embodiments, the contacting occurs *in vitro* or *in vivo.* In some specific embodiments, the contacting occurs inside or outside the cell.

In some specific embodiments, the cell is a eukaryotic cell or a prokaryotic cell.

In some more specific embodiments, the cell is selected from: plant cells, fungal cells, mammalian cells, reptile cells, insect cells, avian cells, fish cells, parasite cells, arthropod cells, invertebrate cells, vertebrate cells, rodent cells, mouse cells, rat cells, primate cells, non-human primate cells, and human cells.

In some more specific embodiments, the contacting results in genome editing.

In some embodiments, the contacting comprises introducing the DEAR nucleic acid manipulation system into the cell.

### EXAMPLES

The present disclosure will be described in further detail below in connection with Detailed Description of Embodiments, and the Examples given are solely for illustrating the present disclosure and not intended to limit its scope. The Examples provided below may serve as a guide for further improvement by those of ordinary skill in the art, and do not in any way constitute a limitation on the present disclosure.

Unless otherwise specified, the experimental methods in the following Examples are all conventional methods, and carried out according to the techniques or conditions described in the literature in the art or according to the product instructions. Unless otherwise specified, the materials, reagents, etc. used in the following Examples are all commercially available.

### Example 1. Screening of RNA sequences of Group IIC introns

In order to screen Group IIC introns, in this Example, 92 Group IIC introns from public databases were utilized, and covariance models of sequence and structure were individually constructed for the RNA sequences of the conserved Domains I-III and V-VI. Moreover, a hidden Markov model for the amino acid sequence characteristics of the potentially existing protein IEP was also constructed. Generally, the length of Group IIC introns does not exceed 4000 nt. Therefore, in this Example, a 4000-bp identification window was set. Potential Group IIC introns needed to be within the 4000-bp range and simultaneously meet the criteria for high-confidence Domains I-III and V-VI. If an IEP protein was not identified in Domain IV, the Group IIC intron was considered an ORF-free Group IIC intron.

Based on the multiple covariance models described above, in this Example, 5,684 Group IIC introns were identified in the global metagenomic dataset. An active Group IIC intron should have multiple highly similar copies in the genome of the same strain. Therefore, in this Example, highly similar candidate Group IIC introns in metagenomes of the same genus were clustered, and a total of 469 potentially active Group IIC introns with multiple copies were identified.

In order to screen stable ORF-free ribozymes, in this Example, candidate Group IIC introns (GII-C introns) were ranked according to the predicted thermal stability of their secondary structures. Moreover, in this Example, RNA secondary structure prediction was also used to further screen candidate Group IIC introns with conserved secondary structures in the target recognition site (TRS).

Furthermore, a Group IIC intron, *Oceanobacillus iheyensis* (O.i.) intron, containing an ORF was obtained based on the literature (N. Toor, K. S. Keating, S. D. Taylor, A. M. Pyle, Crystal structure of a self-spliced group II intron. Science 320, 77-82 (2008).), and its ORF region was deleted to obtain DEAR10 with the sequence as set forth in SEQ ID NO: 57 below.

Finally, DEAR1-DEAR10 were selected as the DEAR nucleic acid manipulation system, and their substrate cleavage activities were verified. Secondary structure predictions for the screened DEAR1-DEAR10 (the RNA secondary structures were predicted using the RNAfold WebServer: http://rna.tbi.univie.ac.at/cgi-bin/RNAWebSuite/RNAfold.cgi) and their domain annotations are shown in FIGS. 1A-1J. As can be seen, the secondary structures of DEAR1-DEAR10 are relatively similar, all comprising Domains I-VI, in which each domain is in the form of a stem-loop structure and naturally separated from each other, and the programmable TRS region is located in the top loop region of Domain I for recognizing nucleic acid substrates. The sequences of DEAR1-DEAR10 are shown in Table 1 below, in which the underlined and bolded part is the TRS.

**Table 1:**

| ID | RNA sequence (5'-3') |
|---|---|
| DEAR1 | GUGCGCUCGGCAUGGGUGCAAUCUCUAGGGUGAAAGUCCCGAACUGCGAAGGCAGAAGUA GCAGUUAGCUUAACGCAAGGGUGUCCGUGGUGACGCGGAAUCUGAAGGAAGCGGGCGGCA AACUUCCGGUCUGAGGAACACGAACUUCAUAUAAGGCUAGGUAUCAUUGGAUGAGUUUGC AAGACAAAACAAAGUCCUUUCUGCCGAAGGUGAUACAGAGUAAAUGAAGCAGAUAGAUGG AAGGAAAGAUUGUACUCUUACCCGAGGAGGUCUGAUGGAUACGUGAAGUGCGCUUCAUAA CCUACUUAGUGAUAAGUAACUGAACCAUCAGAAGUCAGCAGAGGUCAUAGUACGAAUCGG UCUAGAACGAUUCGGAAGGACUGAACAAUCAAGAGAAAAUAGCCCUUGGCAUUCAGUACG UCAUGAUGAACACAGAAAACAUGGUACCUCCCAAGAGAAAGGAAACGGUGAAUCCCGUGG GAAUCUUUUGGAGGGUGGAGUGACGACUGGCAUAAGAAGAUCAGCUAUUUACGGAAGGAA GCUUGCGUCAUUAUCUUGAUUGAACCGCCGUAUACGGAACCGUACGUACGGUGGUGUGAG AGGACGGAGGUUAAUCACCUCCUCCUACUCGAU (SEQ ID NO: 10; Anoxybacillus pushchinoensis) |
| DEAR2 | GUACGACGGGCAUGUCGUACAUCUGAGGUGCAAGUCCUCUGUGGACACCUGCUACCGGUG AACCCAAUAGCGACUCCCAAGCCUGACUAUCGCGAGGUAACGGGGAGAGGAAGGGAUAGC GAAAUCGUGGCUCUACGAACAGAAACGUGAUAUUAAGGCGUAUAUAGUGGAUGAGGGGGC UAGGCACUCUAAAGUCCAAAAAGGUAGUCGUAAUCUAUGUGCGUAAAUCACGAGAGUAAA CGAGGAAAGAUGUACGACUUAUCCCGUGAGGUCUCAUGAGCGUCAAGUAGACGUAGUAAU AACGAAUCAUGAGAAGUCAGCCGAGGUCAUAGUAGUGAUGAAGUUUCUGUAAUGGAAACG |
| | GAGCGAAGGACCGAACGAUUAAAUGAUACCCUAUCUAACUGAUUUUGUCUGCCCUAACAC CUGGUCUGAAAUGACGGUACUCGCAGAACGUAUGCUGAAAGAGGCUGCUCACAAAUAUAG GACGGCACAAGCGGAACGAAAUCAUAUAUAGGUAGGUGUGGGAACAAAAAAAGUGUUCUC GCCAAAGUUUAGUUGAACCGCCGUGUGCCGAACGGCACGCACGGUGGUGUGAGAGGGACU AUCAAUUAGUCCCUACUCGAU (SEQ ID NO: 11; *Streptococcus azizii*) |
| DEAR3 | GUAUGCCCGGCAUGGGCGAUAGCUUUAGGGUGAAAGUCCCGAACAGGGGUUGGCAACACC AACUGUUAGCCAAGAGCAAGGGUGUCCAUCGUGAGGUGGAAUCUGAAAGAAGCUGGAGGC AAAUUCCCGACCUGAGGUACACGAAUCACAUUCGAGGCUGUUAUAUUCGGGCAAGACUGC CAGACAAGUCAAAGCCCUAUGUUGCCAAGGGAUAUAGCAGUAAAUGUGGCAGGUACAUGG GAUGAAAGUUAUCGCUCUUAUCCGGGGAGGUCUGUCGAUCCGGUAAUGGUAAGGAAGUCC UUAACAAUACCAACCCGCAUCGUGAGGUGUCGGCUGAGUCGGCAGAAGUCAGCAGAGGCC AUAGUACUCCUAACGAGGAAGGGCCGAAGGUCAGGGCAGACUAAGAACGGUGCGUUCGAG AGAAUUUGCUGAUAGCAGUUGUCUCUGAAACAGAGACCUAUCUAUAAGAGGGAGUGGUGA AGCCACAAUGGCUUAUAGAAGGGCUGAGAAUUCUCCGGCACAAUACCCGAAAAUCUUAAU CUUAAUCGCUGAGUAAUCAGUUAACCCUGGCCAACCGCCGGAUGCGGACCCGCAUGUCCGG UGGUGUGGGAGGGUCGGCGGAGAUUAUCUCCGCCCCCUAUCCCAAU (SEQ ID NO: 12; Desulfotomaculum_H ferrireducens) |
| DEAR4 | GUGCGCCCAGCAUGGGCGCAAUCUUGGAGGUGAAAGUCCUCCCGUAAGUUGAUCACAGCG AACGAAGCGAAGCGCAACUGCAUGAGGGCGACUGAGUGUGGGGAGGAAGCGUGGAGCGAA GCUGCGAGCCGAUGAACAAGAAUCGGAUAGAAGGCGGUGCCGACCAGGGCGAGCGGGCAA UGAACCGCGAAGCUCUCGUGAUCAAGGGUCAGGCGGCGUAGAUCCGGCGGUUGUGCAGUG AAGGAUUGCGUUCUUACCUGGGGAGAUCUCGCUUUGUGCCUGAAAGGGCGACGGUGUAAG CCGGAGCGAGAAGUCAGCAGAGGUCGUAGUAGUUGGCGGCAUGGCCGGAGAGGCUGGAAG CUGUUGGCGAAGGACCAAAGGAGCAGAAGGGCAAGUCUGCCUGACCAUCCGAAAUGGAUA UGCUUCAGAUGCCCGGAUACGGGGCUCGUUACGAAAAGUGCAGCAGGUGAAGCCUGAAAU ACUUGUGACAGCGAAGAACCAGAUCGGCCGGGUUGGGGACAGGCGAGUCCAGGCGUCAUG UUCGGGUAUGGGCAUGACGACCUCAACCGUUUCAACCGCCCGGUGCGGACCCGCAUGCCGG GUGGUGUGGCAGGGGACCGGUCAGUUAUACUGACCGCCCCUAUGCCGAU (SEQ ID NO: 13; Collimonas pratensis) |
| DEAR5 | GUGCGCCCGGCAUGUACAUGAACUAUAGGGUGUAAGUCCCGAACCCCGAAGACAGAAGUA GAGGUUAGCCAAGAGCAAGGGUGUCCGUGGUGACGCGGAAUCUGAAGGAAGCUGGAGGCA AAACACCGGUCCGAGGAACACGAACCUCAUAUAAGGCUAGGUAUGAUUGAGUGAGUUUGC AUAACAAAACAAAGCUCUUUCUGUCGAAGGUCAUAUCGAGUAAAUGAGGCGGAUAGAUGG UGUGAAAGUGCAUGUACUUACCCGGGGAGGUCUGGCGGAUAUGUGAAGUACUCUUCAUAA CCUACUUAGUGAUAAGUAGCUGAACCGUCAGAAGUCAGCAGAGGUCAUAGUAUUAGUUGG UCUAGAACAACUAAGAAGGACCGAACAAUUAAGAGAGAAUAGCCCUUGGUAUUCAGUGAG UCAUGAUGAACACAGAAAACGUAGUACCUCACUUGAGGGAGGAAGCGGUGAAUCCCGUGG GAGACCUCUUGGAGGGUGGAGUGACCACUGGCAUAAAGAGAACAGCUAUUCACGGAAGUU AUAAAGACUUGCGUCAAUUAUCUUAAUUGAACCGCCGUAUACGGAACCGUACGUACGGUG GUGUGAGAGGACGGGAGUUAAUCGCUCCCUCCUACUCGAU (SEQ ID NO: 14; Bacillus_O smithii) |
| DEAR6 | GUGUGCCCAGUAUGGGCAUCUUUUGUUUUACCGAAAGGUAAAAUAUUAAUCUAGAGGGUG CAAGUCCCUUAUGGGCAGGGGUAACGCCUUGAACCAUUAGUAAGCCGCAAGGGUGGUGAU CGUGAGGUCACAUCUGAAGAAAGCGGGACUACAAAACCCGGUACUGACGAACAGGAACCG |
| | UAUACAGAGGCAUUUAUGCAUGGGUAAGCAACCACAUCAUUGUAAAGCCCUAAGAAUACC AAAAGUGCAUUUAUGUAGAUACGGCAGGGAUCGGGGGAAAGAAGAUGCUCUUACCUGGGG AGGUCUCCAGUACCACGUGUUUGUACAGGAGAAGUCAGCAGAGGUCAUAGUACCUAAGGG AAACGAGUUGCAGCGAAACUGCAUAGGUCUCACAAUUAGGAAAGGACCGAACGUAAUCCU CUUCAAAAUUCGCAUAGGAACACAACUUGUGUAGCCUAUGCUUAAACUAGAAGAUAGUGC CAAGGGUGAAAAGAGCUUUGGUGUGAUGGUUUACGAACCGCCGUAUACGAGACCCGUACG UGCGGUGGUGUGAGGGGUGCACUUCGUCAUUUGAUGGCGGAGCCAUCCACUCGAU (SEQ ID NO: 15; Leeuwenhoekiella) |
| DEAR7 | GUGUGCCCUGAAUGGCAAAUAUAGUUCUUUCAAAUAGAAUAAUCCAGAGAGUGAAAGUCU CUUAUGCGCCUCUUAAAUUGAGGAAGCAUUAGCAAGUCGCAAGGUGGUUUGUCGUGAGAU AUGCACUGAAGUAAGCGAGACUGCAAAGGUCGGUACUGACGUACAGAAACUGUAUAUAGA GGCAUGAUGAAUCGGGUGAGGUAGCACAUCAUACUAAAGCCCCAAGAUUACAAACCAUUC AUCAUGUAGAUGCAGCAGCGAUUGACCGAAGGAUAUUUGUCUUACCGGGGGAGGUCUGUU GAUAGGUUGACUAAGAUUGGCGCGACAAUCGAAGCAACAAUUCCCGAAAGCAAUAGGGAG UUUCAAUAGAAGUCAGCAGAGGUCAUAGUAGUUGGUAGUUACGAGCCGAAGAUAAAUCGG ACGGUCUCACAAGCCAGCGAAGGACUGAAUGUUAAGUCGUUUCUAAUGCAAUAAGGAGCU UUAAGCUUCGCGCUUAGCAGCCUUGUUAUAAAAGAAACAAAGUGCAAUUGGUGAAAAGAG CGAUUGUACUCAUGCUUAACAAACCGCCGUGUACGAGACCCAUACGCACGGUGGUGUGAG GGGCACUCCCCACCUGUUCAUUCAGGUGGGGCUGUCCACUCGAU (SEQ ID NO: 16; PALSA- 967 sp003151745) |
| DEAR8 | GUGCGCCCAGCAUGUGCAAUAGCUAAGCGGUGAAAGUCCGUUGUGGGCCAGGUAGCGGGA ACCACUAGUCGAAGGCAAGGGUGUCCAUCGUGAGGUGGAAUCUGAAGGAAGCCCAAGGCA AAGUCCCGGUCCGAUGAACAAGAACCAGAUAUAAGGCUAAGUGAAGUGGACGAGUUUGCC ACUCAAAACGAAGUCCAACAGCUACCCUGAACUUCACGGAGUAGAUCUGGCGGAUAGAUG GGAUGAAAGUUAUUGCACUUACCUGGGGAGAUCUCAAGAAUAUGCUAUUGAGGCAACCCA UACAGCAAUGUAUGGCUGAAUCUUGAGAAGUCAGCAGAAGUCAUAGUACUCAUUGAGAGA UGAGGAAGGACUGAACAACAGAAGGUUUCGAAUCUUGAAAGAUGCGAAGGGAUACGGAAA AGGCAUACAACUUCGACUAGAAGGUUUCCUACAUGAAGAUAAGGGAGAGCCCGAAAAUAA UGUACCAAGCGCCUAGUAUAACUUCUACAUCUGAAAGAGGGAGAAACGAUGUGAAGGGUC UUAUACAUCAAACCUGUUGAACCGCCGUAUACAGAAUGGUACGUACGGUGGUGCGGAAGG ACGGCAGAUAAAUUAAUUAUCUACCUCCUACUCGAU (SEQ ID NO: 17; Petrotoga mobilis) |
| DEAR9 | GUGCGCCCGGCAUGCGUAUGAACUAUAGGGUGCAAGUCCCGAACCCCGAAGACAGAAGUA GAGGUUAGCCAAGAGCAAGGGUGUCCGUGGCGACGCGGAAUCUGAAGGAAGCUGGAGGCA AAACACCGGUCCGAGGAACACGAAUCUCAUAUAAGGCUAGGUAUGAUUGGAUGAGUUUGC AUUACAAAACAAAGUCCUUUCUGUCGAAGGUCAUAUCGAGUAAAUGAGGCGGAUAGAUGG UGUGAAAGUGCAUACGCUUACCCGGGGAGGUCUGAUGGAAACGUGAAGUACCCUUCAUAA CCUACUUGGUGACAAGUAGCUGAACCAUCAGAAGUCAGCAGAGGUCAUAGUACGAAACUU UGCGUUAUUCAUCUUAAUUGAACCGCCGUAUACGGAACCGUACGUACGGUGGUGUGAGAG GUCGGGAGUUAAUCACUCCCUCCUACUCGAU (SEQ ID NO: 18; Lysinibacillus sphaericus_B) |
| DEAR10 | GUGUGCCCGGCAUGGGUGCAGUCUAUAGGGUGAGAGUCCCGAACUGUGAAGGCAGAAGUA ACAGUUAGCCUAACGCAAGGGUGUCCGUGGCGACAUGGAAUCUGAAGGAAGCGGACGGCA AACCUUCGGUCUGAGGAACACGAACUUCAUAUGAGGCUAGGUAUCAAUGGAUGAGUUUGC AUAACAAAACAAAGUCCUUUCUGCCAAAGUUGGUACAGAGUAAAUGAAGCAGAUUGAUGA AGGGAAAGACUGCAUUCUUACCCGGGGAGGUCUGAUCGAAACGCCAAGCACUCUUGGUAA CCCAUUCAGCAAUGGAUGGCUGAACGGUCAGAAGUCAGCAGAAGUCAUAGUACCCUGCAU |
| | ACUCGAGAAUGUAAGGGGAAGGACGGAACAAUUAAGUUCGCUUAAUUGAACCGCCGUAUA CCGAACGGUACGUACGGUGGUGUGAGAGGACGGGGGUUAGUCGCUCCCUUCUACUCUAU (SEQ ID NO: 57; Oceanobacillus iheyensis) |

Referring to FIGS. 1A-1J, the secondary structures of DEAR1-DEAR10 are as follows:
DEAR1 contains 6 domains (Domains I-VI). Domain I comprises 4 stem-loop/hairpin structures, and a TRS sequence responsible for substrate recognition; Domain II comprises 2 stem-loop/hairpin structures; Domain III comprises 1 stem-loop/hairpin structure; Domain IV comprises 2 stem-loop/hairpin structures; Domain V comprises 1 stem-loop/hairpin structure, which comprises a catalytic core; and Domain VI comprises 1 stem-loop/hairpin structure.
DEAR2 contains 6 domains (Domains I-VI). Domain I comprises 3 stem-loop/hairpin structures, and a TRS sequence responsible for substrate recognition; Domain II comprises 1 stem-loop/hairpin structure; Domain III comprises 1 stem-loop/hairpin structure; Domain IV comprises 4 stem-loop/hairpin structures; Domain V comprises 1 stem-loop/hairpin structure, which comprises a catalytic core; and Domain VI comprises 1 stem-loop/hairpin structure.
DEAR3 contains 6 domains (Domains I-VI). Domain I comprises 4 stem-loop/hairpin structures, and a TRS sequence responsible for substrate recognition; Domain II comprises 2 stem-loop/hairpin structures; Domain III comprises 1 stem-loop/hairpin structure; Domain IV comprises 4 stem-loop/hairpin structures; Domain V comprises 1 stem-loop/hairpin structure, which comprises a catalytic core; and Domain VI comprises 1 stem-loop/hairpin structure.
DEAR4 contains 6 domains (Domains I-VI). Domain I comprises 6 stem-loop/hairpin structures, and a TRS sequence responsible for substrate recognition; Domain II comprises 3 stem-loop/hairpin structures; Domain III comprises 1 stem-loop/hairpin structure; Domain IV comprises 3 stem-loop/hairpin structures; Domain V comprises 1 stem-loop/hairpin structure, which comprises a catalytic core; and Domain VI comprises 1 stem-loop/hairpin structure.
DEAR5 contains 6 domains (Domains I-VI). Domain I comprises 4 stem-loop/hairpin structures, and a TRS sequence responsible for substrate recognition; Domain II comprises 2 stem-loop/hairpin structures; Domain III comprises 1 stem-loop/hairpin structure; Domain IV comprises 2 stem-loop/hairpin structures; Domain V comprises 1 stem-loop/hairpin structure, which comprises a catalytic core; and Domain VI comprises 1 stem-loop/hairpin structure.
DEAR6 contains 6 domains (Domains I-VI). Domain I comprises 6 stem-loop/hairpin structures, and a TRS sequence responsible for substrate recognition; Domain II comprises 1 stem-loop/hairpin structure; Domain III comprises 1 stem-loop/hairpin structure; Domain IV comprises 3 stem-loop/hairpin structures; Domain V comprises 1 stem-loop/hairpin structure, which comprises a catalytic core; and Domain VI comprises 1 stem-loop/hairpin structure.
DEAR7 contains 6 domains (Domains I-VI). Domain I comprises 4 stem-loop/hairpin structures, and a TRS sequence responsible for substrate recognition; Domain II comprises 2 stem-loop/hairpin structures; Domain III comprises 1 stem-loop/hairpin structure; Domain IV comprises 2 stem-loop/hairpin structures; Domain V comprises 1 stem-loop/hairpin structure, which comprises a catalytic core; and Domain VI comprises 1 stem-loop/hairpin structure.
DEAR8 contains 6 domains (Domains I-VI). Domain I comprises 5 stem-loop/hairpin structures, and a TRS sequence responsible for substrate recognition; Domain II comprises 2 stem-loop/hairpin structures; Domain III comprises 1 stem-loop/hairpin structure; Domain IV comprises 3 stem-loop/hairpin structures; Domain V comprises 1 stem-loop/hairpin structure, which comprises a catalytic core; and Domain VI comprises 1 stem-loop/hairpin structure.
DEAR9 contains 6 domains (Domains I-VI). Domain I comprises 4 stem-loop/hairpin structures, and a TRS sequence responsible for substrate recognition; Domain II comprises 2 stem-loop/hairpin structures; Domain III comprises 1 stem-loop/hairpin structure; Domain IV comprises 1 stem-loop/hairpin structure; Domain V comprises 1 stem-loop/hairpin structure, which comprises a catalytic core; and Domain VI comprises 1 stem-loop/hairpin structure.
DEAR10 contains 6 domains (Domains I-VI). Domain I comprises 4 stem-loop/hairpin structures, and a TRS sequence responsible for substrate recognition; Domain II comprises 2 stem-loop/hairpin structures; Domain III comprises 1 stem-loop/hairpin structure; Domain IV comprises 1 stem-loop/hairpin structure, and a 1260-nt ORF region (deleted in DEAR10); Domain V comprises 1 stem-loop/hairpin structure, which comprises a catalytic core; and Domain VI comprises 1 stem-loop/hairpin structure.

The nucleotides corresponding to D1-D6 in DEAR1-DEAR10 are shown below:
**DEAR1**
   D1: 1-266, D2: 267-339, D3: 340-385, D4: 386-562, D5: 563-596, D6: 597-633, and
   TRS: 181-186
**DEAR2**
   D1: 1-267, D2: 268-318, D3: 319-375, D4: 376-562, D5: 563-596, D6: 590-621, and
   TRS: 181-186
      Or,
**DEAR2**
   D1: 1-267, D2: 268-321, D3: 322-375, D4: 376-555, D5: 556-589, D6: 590-621, and
   TRS: 181-186
**DEAR3**
   D1: 1-266, D2: 267-350, D3: 351-390, D4: 391-572, D5: 573-606, D6: 607-647, and
   TRS: 181-186
      Or,
**DEAR3**
   D1: 1-266, D2: 267-353, D3: 354-388, D4: 389-572, D5: 573-606, D6: 607-647, and
   TRS: 181-186
**DEAR4**
   D1: 1-262, D2: 263-318, D3: 319-379, D4: 380-573, D5: 574-607, D6: 608-650, and
   TRS: 179-184
**DEAR5**
   D1: 1-266, D2: 267-342, D3: 343-385, D4: 386-569, D5: 570-603, D6: 604-640, and
   TRS: 181-186
      Or,
**DEAR5**
   D1: 1-266, D2: 267-339, D3: 340-385, D4: 386-569, D5: 570-603, D6: 604-640, and
   TRS: 181-186
**DEAR6**
   D1: 1-299, D2: 300-342, D3: 343-411, D4: 412-515, D5: 516-550, D6: 551-595, and
   TRS: 214-219
      Or,
**DEAR6**
   D1: 1-299, D2: 300-339, D3: 340-411, D4: 412-515, D5: 516-550, D6: 551-595, and
   TRS: 214-219
**DEAR7**
   D1: 1-290, D2: 291-380, D3: 381-448, D4: 449-561, D5: 562-596, D6: 597-644, and
   TRS: 206-211
      Or,
**DEAR7**
   D1: 1-290, D2: 291-377, D3: 378-449, D4: 450-561, D5: 562-596, D6: 597-644, and
   TRS: 206-211
**DEAR8**
   D1: 1-267, D2: 268-338, D3: 339-375, D4: 376-613, D5: 614-594, D6: 595-636, and
   TRS: 180-185
      Or,
**DEAR8**
   D1: 1-267, D2: 268-335, D3: 336-375, D4: 376-559, D5: 560-593, D6: 594-636, and
   TRS: 180-185
**DEAR9 (without Domain D4, represented by** "---")
   D1: 1-266, D2: 267-342, D3: 343-380, D4: ---, D5: 381-415, D6: 416-451, and
   TRS: 181-186
      Or,
**DEAR9 (without Domain D4, represented by "---")**
   D1: 1-266, D2: 267-339, D3: 340-380, D4: ---, D5: 381-414, D6: 415-451, and
   TRS: 181-186
**DEAR10**
   D1: 1-266, D2: 267-339, D3: 340-388, D4: 389-408, D5: 409-442, D6: 443-479, and
   TRS: 181-186

Illustratively, Domain I (D1) in DEAR1 corresponds to nucleotide 1 to nucleotide 266 of the nucleotide sequence of DEAR1 (SEQ ID NO: 1).

It should be noted that for the domain division of the primary sequences described above, the position of each domain was determined by a three-dimensional structure for DEAR1, DEAR2, DEAR3, DEAR5, and DEAR6, and the position of each domain was confirmed by performing sequence alignment with DEAR1, DEAR2, DEAR3, DEAR5, and DEAR6 using Clustal (F. Sievers et al., Fast, scalable generation of high-quality protein multiple sequence alignments using Clustal Omega. Mol Syst Biol 7, 539 (2011). doi:10.1038/msb.2011.75) for the remaining DEARs (DEAR4 and DEAR7-DEAR10). Due to the plasticity of the RNA structure, there was a degree of deviation in the sequence boundaries of each domain (e.g., about -20-+20 nt). Moreover, the boundary division of the secondary domain was the same as that of the primary structure. The secondary structure prediction was performed using the RNAfold (R. Lorenz et al., ViennaRNA Package 2.0. Algorithms Mol Biol 6, 26 (2011). doi:10.1186/1748-7188-6-26) method. Due to the dynamic nature of the RNA structure, each RNA domain may exhibit a deviation of 0-5 domains.

Therefore, there may be different division ways for the positions of the nucleotides corresponding to D1-D6 in DEAR1-DEAR10 above. Furthermore, for example, in the secondary structure prediction of DEAR9, Domain IV contains 1 stem-loop/hairpin structure; however, the corresponding primary sequence is absent in the primary structure as confirmed by sequence alignment with DEAR1, DEAR2, DEAR3, DEAR5, and DEAR6 using Clustal, which is also reasonable.

### Example 2. Preparation method of RNAs

The corresponding DNA sequences of DEAR1-DEAR10 screened in Example 1 were first synthesized, and the T7 promoter (TAATACGACTCACTATA; SEQ ID NO: 19) was added upstream of each DEAR via PCR. The PCR amplification product was purified using DNA purification magnetic beads (VAHTS DNA Clean Beads, Vazyme, Cat. No. N411-01), and the purified product was used as the template for *in vitro* transcription (IVT). The *in vitro* transcription reaction was performed in 30 mM Tris pH 8.1, 25 mM MgCl₂, 0.01% Triton X-100, 2 mM spermidine, and 5 mM DTT, with each NTP added at 5 mM. RNAse inhibitor (Promega, Cat. No. N2111) and T7 RNA polymerase (NEB, Cat. No. M0251S) were added according to the instructions of the reagent suppliers. After 4 hours of reaction at 37°C, DNase I (Promega, Cat. No. M6101) digestion and proteinase K (Beyotime, Cat. No. ST533) digestion were carried out sequentially to remove the DNA template and proteins. Then, the transcription product was washed and concentrated using a concentrator with a molecular weight cutoff of 100 kDa. Electrophoresis was performed using 8% Urea-PAGE to detect the mass of RNA. The specific results are shown in FIGS. 2A and 2B. As can be seen, RNAs of 10 ribozymes were successfully prepared. When compared with RNAs of known lengths, it was found that the size of each RNA ribozyme matched its theoretical length.

### Example 3. In Vitro cleavage of single-stranded RNAs, single-stranded DNAs, and plasmids by DEAR nucleic acid manipulation systems

### 1. In Vitro cleavage of single-stranded RNAs by DEAR nucleic acid manipulation systems

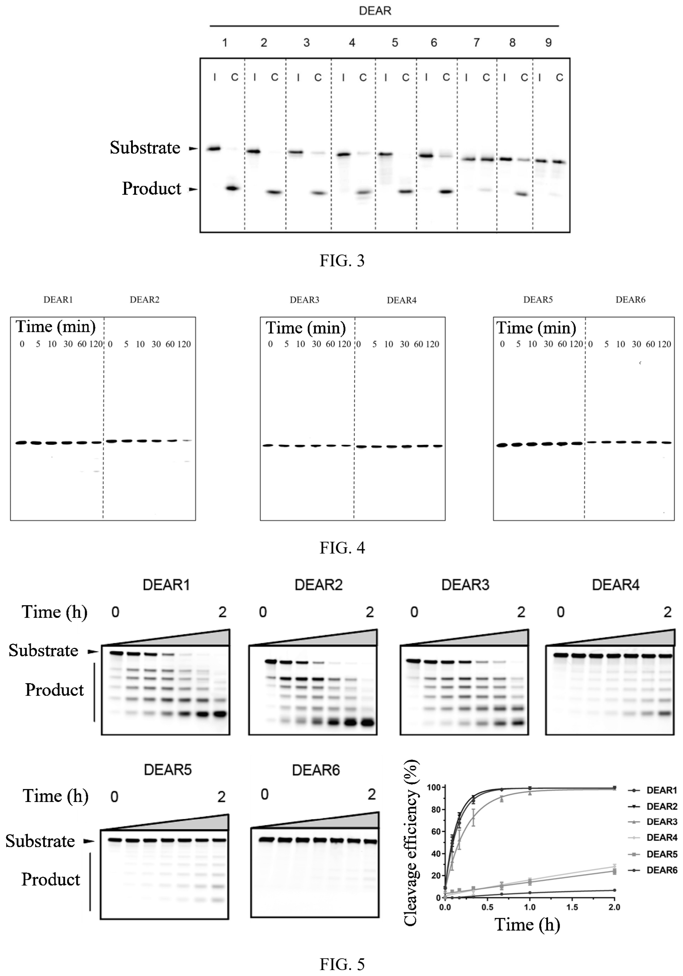

Single-stranded RNA (ssRNA) substrates with the corresponding DEAR target sequences (in which the underlined and bolded part is the target sequence recognized by DEAR) were synthesized according to the sequences shown in Table 2 below, and each single-stranded RNA substrate was labeled with -Cy5 at its 3' end. Each DEAR (1.5 µM) was individually incubated with the corresponding single-stranded RNA substrate (100 nM) under conditions of 500 mM NH₄Cl, 125 mM MgCl₂, 40 mM MOPS 7.5, and 50°C for 1 h. After the reaction was terminated, Urea-PAGE electrophoresis was performed, and the gel fluorescence signals were scanned on a fluorescence imager. The results are shown in FIG. 3. As shown in FIG. 3, the cleavage products of ssRNA are located in the lower part, and it can be seen that all DEAR1-DEAR9 can cleave the single-stranded RNA. In FIG. 3, I represents the input ssRNA, and C represents the cleavage product.

**Table 2:**

| ID # | SEQ ID NO: | Sequences (5'-3'; in which the underlined and bolded part is the target sequence) |
|---|---|---|
| RNA-DEAR1 | SEQ ID NO: 20 | ACCAAA**UGUCUU**AGCGGAUCGGAU |
| RNA-DEAR2 | SEQ ID NO: 21 | GAGAAA**UGCCUAC**AACACACCACC |
| RNA-DEAR3 | SEQ ID NO: 22 | GUAACA**UGUCUG**CAAAACCUCCAA |
| RNA-DEAR4 | SEQ ID NO: 23 | GGAGGC**UUCAUU**AACGGCAACAGA |
| RNA-DEAR5 | SEQ ID NO: 24 | ACCCAC**UGUUAU**CCGACGACGAGC |
| RNA-DEAR6 | SEQ ID NO: 25 | CUUCAC**UGAUGU**ACAACCAAGAGA |
| RNA-DEAR7 | SEQ ID NO: 25 | CUUCAC**UGAUGU**ACAACCAAGAGA |
| RNA-DEAR8 | SEQ ID NO: 27 | GUAACA**UGAGUG**CAAAACCUCCAA |
| RNA-DEAR9 | SEQ ID NO: 28 | ACCCAC**UGUAAU**CCGACGACGAGC |

### 2. Verification of ssRNA target regions for DEAR nucleic acid manipulation systems

DEAR1-DEAR6 (each at 1.5 µM) were individually incubated with the corresponding single-stranded RNA substrate (100 nM) that cannot pair with the TRS region (the substrate used for DEAR1 was the sequence as set forth in SEQ ID NO: 21; the substrate used for DEAR2 was the sequence as set forth in SEQ ID NO: 23; the substrate used for DEAR3 was the sequence as set forth in SEQ ID NO: 23; the substrate used for DEAR4 was the sequence as set forth in SEQ ID NO: 22; the substrate used for DEAR5 was the sequence as set forth in SEQ ID NO: 21; and the substrate used for DEAR6 was the sequence as set forth in SEQ ID NO: 23) under conditions of 10 mM KCl, 50 mM MgCl₂, 40 mM MOPS 7.5, and 37°C. Samples were taken at given time points (0 min, 5 min, 10 min, 30 min, 60 min, and 120 min). After the reaction was terminated, Urea-PAGE electrophoresis was performed, and the gel fluorescence signals were scanned on a fluorescence imager. The results of the gel images are shown in FIG. 4. As shown in FIG. 4, the cleavage products of ssRNA are located below the substrates, and when the substrate cannot pair with the TRS region, it cannot be cleaved.

### 3. In Vitro cleavage of single-stranded DNAs by DEAR nucleic acid manipulation systems

Single-stranded DNA (ssDNA) substrates with the corresponding target sequences for DEAR1-DEAR6 (in which the underlined and bolded part is the target sequence recognized by DEAR) were individually synthesized according to the sequences shown in Table 3 below, and were labeled with -Cy5 at the 3' end. Subsequently, each DEAR (1.5 µM) was incubated with the corresponding single-stranded DNA substrate (100 nM) under conditions of 500 mM NH₄Cl, 125 mM MgCl₂, 40 mM MOPS 7.5, and 50°C. Samples were taken at given time points (0 min, 5 min, 10 min, 20 min, 40 min, 60 min, and 120 min, shown as 0-2 h in the figure). After the reaction was terminated, Urea-PAGE electrophoresis was performed, and the gel fluorescence signals were scanned on a fluorescence imager. The results of the gel images and the efficiency curve are shown in FIG. 5. As shown in FIG. 5, the cleavage products of ssDNA are located below the substrates, and it can be seen that all DEAR1-DEAR6 can cleave the single-stranded DNA.

**Table 3:**

| ID # | SEQ ID NO: | Sequences (5'-3'; in which the underlined and bolded part is the target sequence) |
|---|---|---|
| 5X-DEAR1 | SEQ ID NO: 29 | TAGAGAACTT TGTCTT TGTCTT TGTCTT TGTCTT TGTCTT TTTACTGGGTAGTGCGTAGG |
| 5X-DEAR2 | SEQ ID NO: 30 | TAGAGAACTT TGCCTA TGCCTA TGCCTA TGCCTA TGCCTA TTTACTGGGTAGTGCGTAGG |
| 5X-DEAR3 | SEQ ID NO: 31 | TAGAGAACTT TGTCTG TGTCTG TGTCTG TGTCTG TGTCTG TTTACTGGGTAGTGCGTAGG |
| 5X-DEAR4 | SEQ ID NO: 32 | TAGAGAACTT TTCATT TTCATT TTCATT TTCATT TTCATT TTTACTGGGTAGTGCGTAGG |
| 5X-DEAR5 | SEQ ID NO: 33 | TAGAGAACTT TGTTAT TGTTAT TGTTAT TGTTAT TGTTAT TTTACTGGGTAGTGCGTAGG |
| 5X-DEAR6 | SEQ ID NO: 34 | TAGAGAACTT TGATGT TGATGT TGATGT TGATGT TGATGT TTTACTGGGTAGTGCGTAGG |

### 4. Verification of ssDNA target regions for DEAR nucleic acid manipulation systems

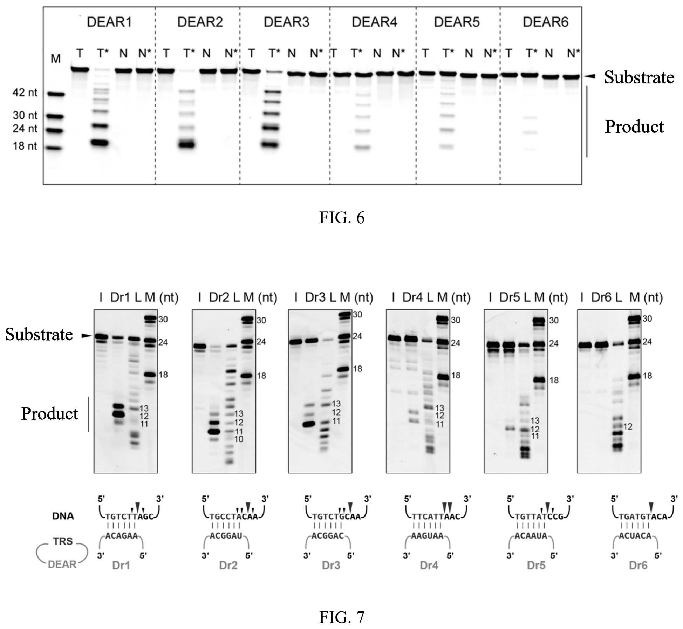

DEAR1-DEAR6 (1.5 µM) were each individually incubated with the corresponding single-stranded DNA substrate (100 nM) that can or cannot pair with the TRS region (the substrates used for DEAR1 were the sequences as set forth in SEQ ID NO: 29 and SEQ ID NO: 30, respectively; the substrates used for DEAR2 were the sequences as set forth in SEQ ID NO: 30 and SEQ ID NO: 32, respectively; the substrates used for DEAR3 were the sequences as set forth in SEQ ID NO: 31 and SEQ ID NO: 32, respectively; the substrates used for DEAR4 were the sequences as set forth in SEQ ID NO: 32 and SEQ ID NO: 31, respectively; the substrates used for DEAR5 were the sequences as set forth in SEQ ID NO: 33 and SEQ ID NO: 30, respectively; and the substrates used for DEAR6 were the sequences as set forth in SEQ ID NO: 34 and SEQ ID NO: 32, respectively) under conditions of 500 mM NH₄Cl, 125 mM MgCl₂, 40 mM MOPS 7.5, and 50°C for 1 h. After the reaction was terminated, Urea-PAGE electrophoresis was performed, and the gel fluorescence signals were scanned on a fluorescence imager. The results of the gel images are shown in FIG. 6. As shown in FIG. 6, in which T represents the paired substrate, T* represents the cleavage product of the paired substrate, N represents the unpaired substrate, N* represents the cleavage product of the unpaired substrate, and M represents the marker, the cleavage products of ssDNA are located below the substrates, and it can be seen that all DEAR1-DEAR6 can cleave the single-stranded DNA, and when the substrate cannot pair with the TRS region, it cannot be cleaved.

### 5. Verification of ssDNA cleavage sites for DEAR nucleic acid manipulation systems

Single-stranded DNA (ssDNA) substrates with the corresponding target sequences for DEAR1-DEAR6 (in which the underlined and bolded part is the target sequence recognized by DEAR) were synthesized according to the sequences shown in Table 4 below, and were labeled with -Cy5 at the 3' end. Subsequently, each DEAR (1.5 µM) was incubated with the corresponding single-stranded DNA substrate (100 nM) under conditions of 500 mM NH₄Cl, 125 mM MgCl₂, 40 mM MOPS 7.5, and 50°C for 24 h. After the reaction was terminated, Urea-PAGE electrophoresis was performed, and the gel fluorescence signals were scanned on a fluorescence imager. The gel images are shown in FIG. 7. As shown in FIG. 7, in which the larger triangle represents the major cleavage site, the smaller triangle represents the minor cleavage site, I represents the input substrate, Dr1-Dr6 represent the corresponding cleavage products of DEAR1-DEAR6, L represents the ladder produced by random digestion of ssDNA using DNase I (Promega, Cat. No. M6101) to indicate the length of the product, and M represents the marker, the cleavage products of ssDNA are located below the substrates, and it can be seen that the major cleavage site is located 0-1 nt downstream of the 3' end of the region paired with TRS.

**Table 4:**

| ID # | SEQ ID NO: | Sequences (5'-3'; in which the underlined and bolded part is the target sequence) |
|---|---|---|
| 1X-DEAR1 | SEQ ID NO: 35 | ACCAAA**TGTCTT**AGCGGATCGGAT |
| 1X-DEAR2 | SEQ ID NO: 36 | GAGAAA**TGCCTA**CAACACACCACC |
| 1X-DEAR3 | SEQ ID NO: 37 | GTAACA**TGTCTG**CAAAACCTCCAA |
| 1X-DEAR4 | SEQ ID NO: 38 | GGAGGC**TTCATT**AACGGCAACAGA |
| 1X-DEAR5 | SEQ ID NO: 39 | ACCCAC**TGTTAT**CCGACGACGAGC |
| 1X-DEAR6 | SEQ ID NO: 40 | CTTCAC**TGATGT**ACAACCAAGAGA |

### 6. Optimization of DNA cleavage conditions for DEAR1

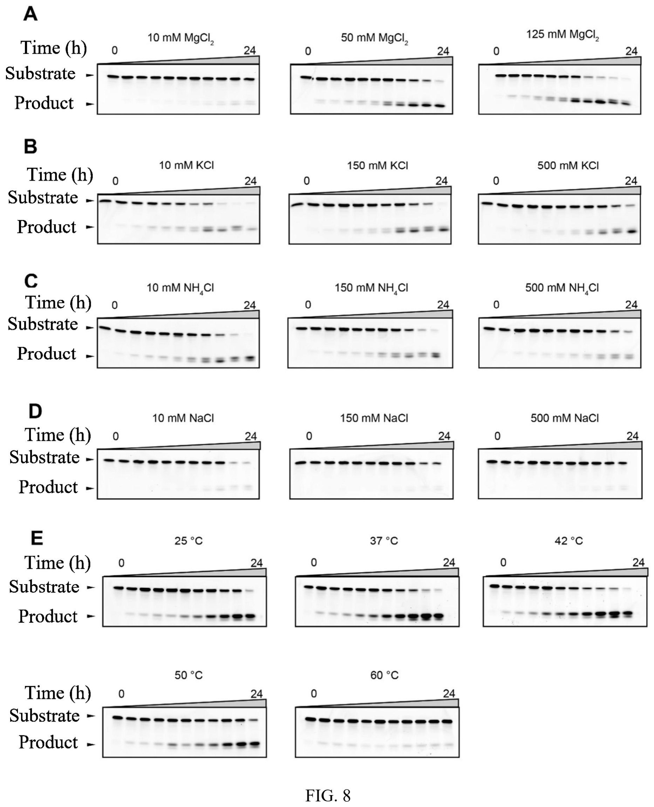

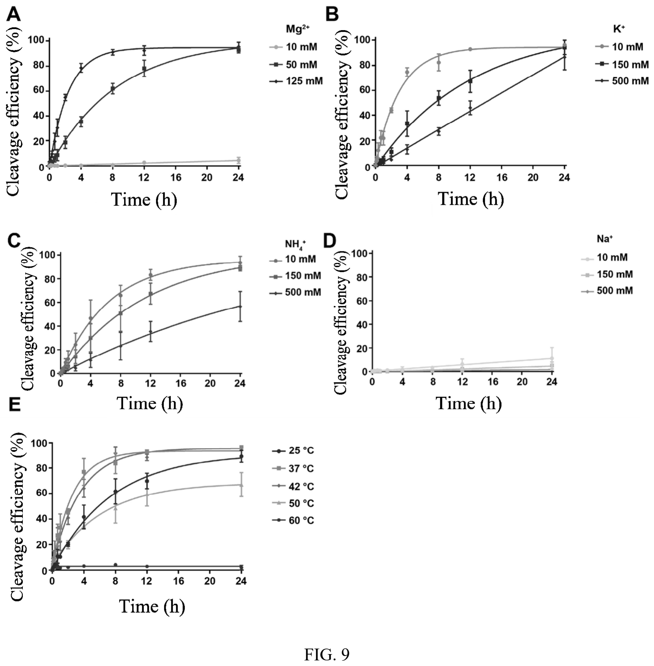

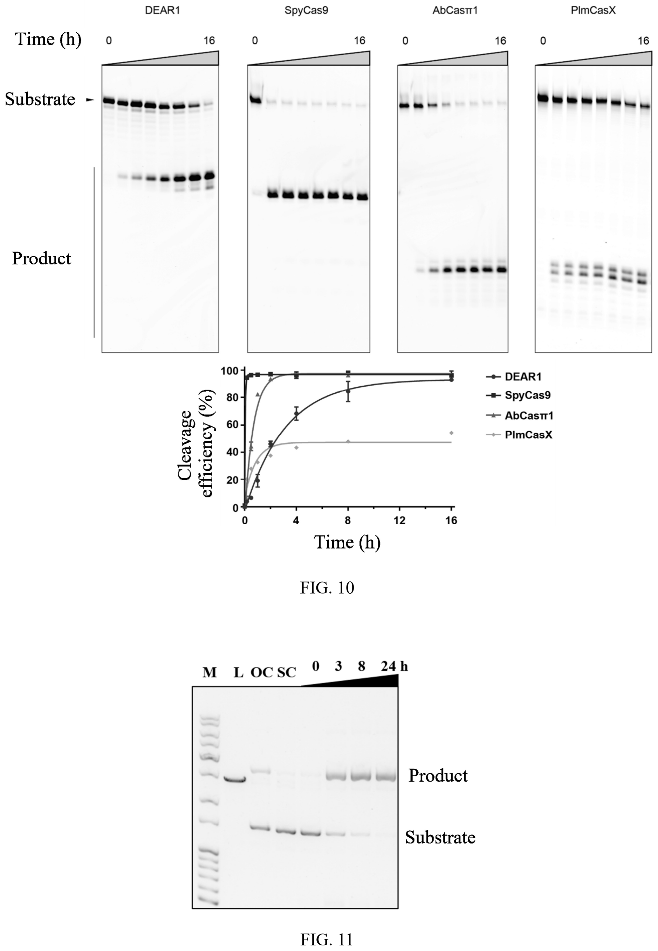

DEAR1 (1.5 µM) was incubated with the single-stranded DNA 1X-DEAR1 substrate (SEQ ID NO: 35; 100 nM) under conditions of various concentrations of monovalent and divalent ions and different temperatures. Samples were taken at given time points (0 min, 5 min, 10 min, 20 min, 40 min, 1 h, 2 h, 4 h, 8 h, 12 h, and 24 h). After the reaction was terminated, Urea-PAGE electrophoresis was performed, and the gel fluorescence signals were scanned on a fluorescence imager. The results of the gel images and efficiency curves are shown in FIGS. 8-9. As shown in FIGS. 8-9, the cleavage products of DNA are located below the substrate, and it can be seen that DEAR1 prefers K⁺, and the lower the concentration the more active it is, which is different from the Group II introns previously reported (N. Toor, K. S. Keating, S. D. Taylor, A. M. Pyle, Crystal structure of a self-spliced group II intron. Science 320, 77-82 (2008); and C. Quiroga, P. H. Roy, D. Centron, The S.ma.I2 class C group II intron inserts at integron attC sites. Microbiology (Reading) 154, 1341-1353 (2008).). In addition, DEAR1 is more active at higher Mg²⁺ concentrations. It is active at 25-50°C, and the activity is relatively high at a temperature of 37-42°C. Specific reaction conditions: FIG. 8A, 150 mM KCl, 10/50/125 mM MgCl₂, 40 mM MOPS 7.5, and 37°C; FIG. 8B, 10/150/500 mM KCl, 50 mM MgCl₂, 40 mM MOPS 7.5, and 37°C; FIG. 8C, 10/150/500 mM NH₄Cl, 50 mM MgCl₂, 40 mM MOPS 7.5, and 37°C; FIG. 8D, 10/150/500 mM NaCl, 50 mM MgCl₂, 40 mM MOPS 7.5, and 37°C; and FIG. 8E, 10 mM KCl, 50 mM MgCl₂, 40 mM MOPS 7.5, and 25/37/42/50/60°C.

### 7. Comparison of DNA cleavage efficiency of DEAR1 and RNA-guided proteases

DEAR1 (1.5 µM) was incubated with the single-stranded DNA 1X-DEAR1 substrate (SEQ ID NO: 35; 100 nM) under conditions of 10 mM KCl, 50 mM MgCl₂, 40 mM MOPS 7.5, and 37°C. Samples were taken at given time points (0 min, 10 min, 30 min, 1 h, 2 h, 4 h, 8 h, and 16 h). For the CRISPR-Cas nuclease systems used, the reaction systems were prepared at a ratio of RNP : DNA = 15 : 1 according to the methods described in A. Sun et al., The compact Caspi (Cas121) 'bracelet' provides a unique structural platform for DNA manipulation. Cell Res 33, 229-244 (2023), C. A. Tsuchida et al., Chimeric CRISPR-CasX enzymes and guide RNAs for improved genome editing activity. Mol Cell 82, 1199-1209 e1196 (2022), and M. Jinek et al., A programmable dual-RNA-guided DNA endonuclease in adaptive bacterial immunity. Science 337, 816-821 (2012). Samples were taken at given time points (0 min, 10 min, 30 min, 1 h, 2 h, 4 h, 8 h, and 16 h). After the reaction was terminated, Urea-PAGE electrophoresis was performed, and the gel fluorescence signals were scanned on a fluorescence imager. The results of the gel images and the efficiency curve are shown in FIG. 10. As shown in FIG. 10, the cleavage products of DNA are located below the substrate, and it can be seen that the cleavage efficiency of DEAR1 is close to that of SpyCas9 and AbCasπ1, and higher than that of PlmCasX.

### 8. In Vitro cleavage of plasmids by DEAR nucleic acid manipulation system

A plasmid containing the corresponding target sequence for DEAR1 (TGTCTTAAGACA; SEQ ID NO: 41) was designed and synthesized (using the commercially available pUC19 plasmid from Addgene (Plasmid #50005) as the backbone). DEAR1 (1.5 µM) was incubated with the plasmid substrate (0.03 µM) under conditions of 150 mM KCl, 10 mM MgCl₂, 40 mM MOPS 7.5, and 37°C. Samples were taken at given time points (0 h, 3 h, 8 h, and 24 h). After the reaction was terminated, agarose gel electrophoresis was performed, and the gel image was obtained by taking a photograph on an ultraviolet imager. The results are shown in FIG. 11, in which L represents the plasmid treated with EcoRI (NEB, Cat. No. R0101V) in a linear double-stranded state; OC represents the plasmid treated with Nt.BspQI (NEB, Cat. No. R0644S) in an open circular state; SC represents the untreated plasmid in a supercoiled state; and 0, 3, 8, and 24 represent the time for the plasmid to be cleaved with DEAR1 (in hours). Referring to FIG. 11, the substrate plasmids exist in a supercoiled state, and the products resulting from the cleavage reaction appear in an open circular state and are located above the substrate. It can be seen that DEAR1 can cleave the plasmid.

### 9. In Vitro cleavage of single-stranded RNAs by DEAR10

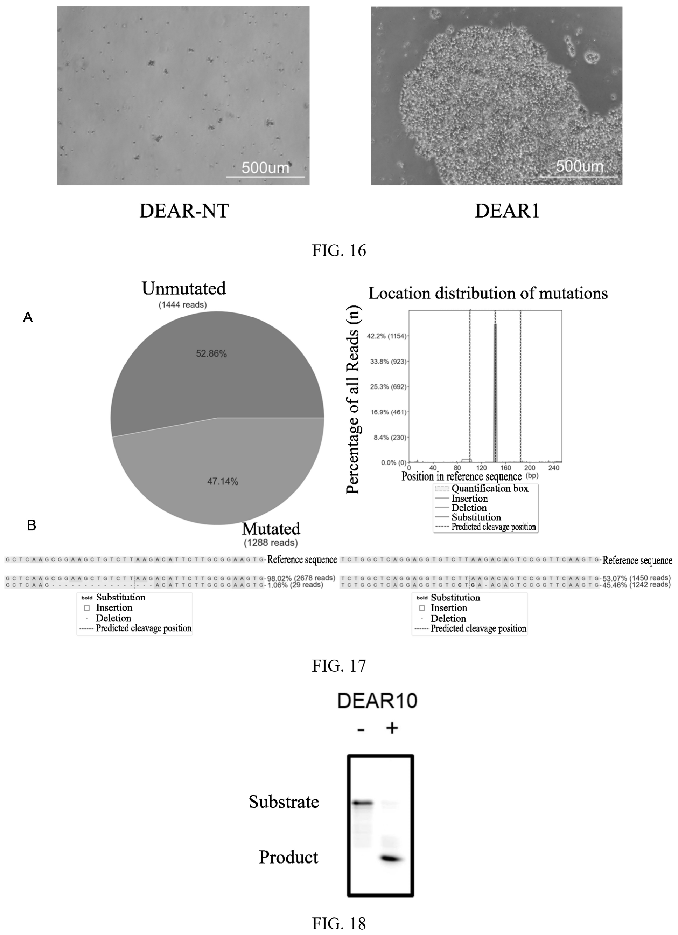

The RNA substrate sequence (RNA-DEAR10) used in this experiment:
ACCCACUGUUAUCCGACGACGAGC (the same as RNA-DEAR5, SEQ ID NO: 24)
DEAR10 RNA (1.5 µM) was incubated with the single-stranded RNA substrate (100 nM, SEQ ID NO: 24) under conditions of 500 mM NH₄Cl, 125 mM MgCl₂, 40 mM MOPS 7.5, and 50°C for 1 h. After the reaction was terminated, Urea-PAGE electrophoresis was performed, and the gel fluorescence signals were scanned on a fluorescence imager. The results are shown in FIG. 18. As shown in FIG. 18, the cleavage product of ssRNA is located in the lower part, and it can be seen that DEAR10 can cleave the single-stranded RNA.

### 10. In Vitro cleavage of single-stranded DNAs by DEAR10

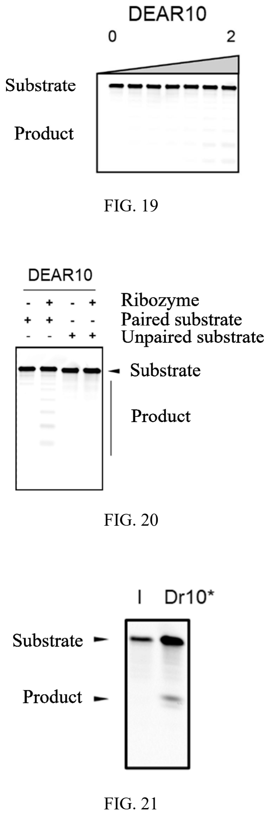

The DNA substrate sequence (5X-DEAR10) used in this experiment:
TAGAGAACTT TGTTAT TTTACTGGGTAGTGCGTAGG (the same as 5X-DEAR5, SEQ ID NO: 33)
DEAR10 RNA (1.5 µM) was incubated with the corresponding single-stranded DNA substrate (100 nM, SEQ ID NO: 33) under conditions of 500 mM NH₄Cl, 125 mM MgCl₂, 40 mM MOPS 7.5, and 50°C. Samples were taken at given time points (0 min, 5 min, 10 min, 20 min, 40 min, 60 min, and 120 min, shown as 0-2 h in the figure). After the reaction was terminated, Urea-PAGE electrophoresis was performed, and the gel fluorescence signals were scanned on a fluorescence imager. The results of the gel image are shown in FIG. 19. As shown in FIG. 19, the cleavage products of ssDNA are located below the substrate, and it can be seen that DEAR10 can cleave the single-stranded DNA.

### 11. Verification of ssDNA target regions for DEAR10

DEAR10 (1.5 µM) was individually incubated with the corresponding single-stranded DNA substrate (100 nM) that can or cannot pair with its TRS region (the sequences as set forth in SEQ ID NO: 33 and SEQ ID NO: 30, respectively) under conditions of 500 mM NH₄Cl, 125 mM MgCl₂, 40 mM MOPS 7.5, and 50°C for 1 h. After the reaction was terminated, Urea-PAGE electrophoresis was performed, and the gel fluorescence signals were scanned on a fluorescence imager. The results of the gel image are shown in FIG. 20, in which the cleavage products of ssDNA are located below the substrates, and it can be seen that DEAR10 can cleave the single-stranded DNA, and when the substrate cannot pair with the TRS region, it cannot be cleaved.

### Example 4. Plasmid interference in E. coli cells

### 1. Construction of target plasmids

A ccdB toxin gene-inducible expression plasmid (Addgene, Plasmid ID: 69056) containing the corresponding target sequences for DEAR1-DEAR3 in the plasmid replication origin (ori) was used as the target plasmid.

### 2. Construction of DEAR expression plasmids

In the DEAR expression plasmids, the J23119 promoter (with the specific sequence of (SEQ ID NO: 42): TTGACAGCTAGCTCAGTCCTAGGTATAATACTAGT) was used to drive the expression of each DEAR sequence. The construction method was as follows: The J23119 promoter was ligated to each of DEAR1-DEAR3. Then, the sequences of DEAR1-DEAR3, each ligated to the J23119 promoter were individually inserted into the pCDFDuet1 plasmid vector (Novagen, Cat. No. 71340-3) via homologous recombination, thereby individually replacing the entire sequence of the 410-3765 region of this plasmid.

### 3. Construction of CRISPR-Cas nuclease system

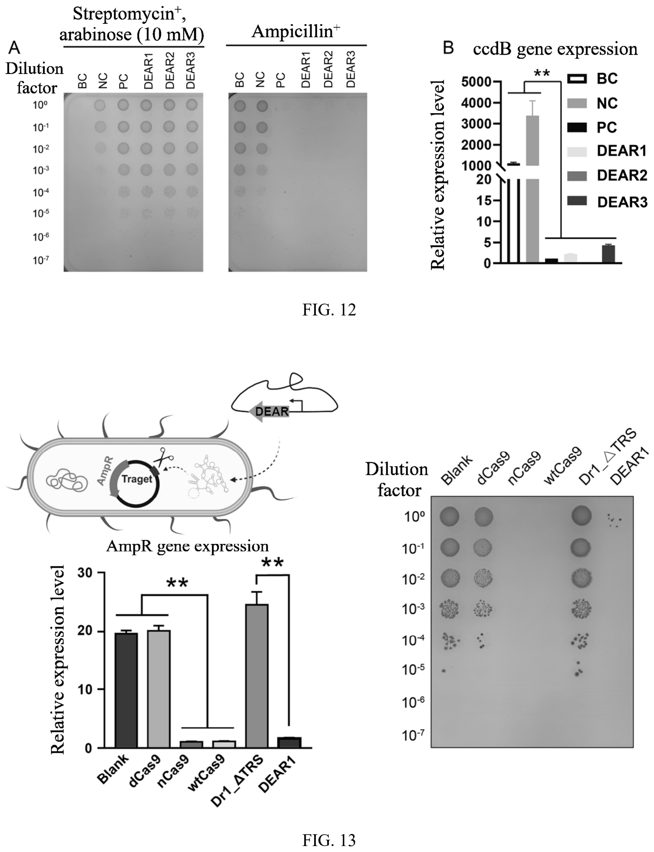

In the CRISPR-Cas nuclease expression plasmids, the Trc promoter (with the specific sequence of (SEQ ID NO: 43): TTGACAATTAATCATCCGGCTCGTATAATG) was used to drive the expression of the Cas9 nuclease, and the J23119 promoter (with the specific sequence as above) was used to drive the expression of the corresponding guide RNA (sgRNA) sequence. The sgRNA expressed in the positive control group (labeled as PC in FIG. 12, i.e., the PC group) contained a 20-base target sequence (with the specific sequence of (SEQ ID NO: 44): GCGATAAGTCGTGTCTTACC), and the target plasmid was cleaved as guided by the sgRNA; and the sgRNA expressed in the negative control group (labeled as NC in FIG. 12, i.e., the NC group) did not contain a 20-base target sequence, and the target plasmid could not be cleaved. The construction method was as follows: The Trc promoter was ligated to the Cas9 sequence, followed by the J23119 promoter and the sgRNA. Then, the entire sequence of Trc-Cas9-J23119-sgRNA was inserted into the pCDFDuet1 plasmid vector (Novagen, Cat. No. 71340-3) via homologous recombination, thereby replacing the entire sequence of the 410-3765 region of this plasmid.

### 4. Detection of plasmid interference in E. coli cells

The target plasmid from Step 1 was co-transformed into the *E. coli* strain BW25141 (CGSC, Strain Accession No.: 7635) with the different expression plasmids constructed in Steps 2 and 3 (the DEAR1-DEAR3 expression plasmids and the CRISPR-Cas nuclease system expression plasmids), respectively. After culturing for a certain period, bacterial culture samples were taken and individually cultured on plates containing a ccdB inducer (10 mM arabinose; Sangon, Cat. No. A610071) and plates containing the antibiotic (ampicillin) against which the target plasmid was resistant. Referring to FIG. 12A, when the bacteria contained only the target plasmid, they can survive on the ampicillin plates and colonies were formed, while they cannot grow on the ccdB induction expression plates (the BC group), which was essentially consistent with the survival/death situation of the bacterial colonies in which the transformed expression plasmid did not mediate the cleavage of the target plasmid (the NC group, in which Cas9 was expressed and ccdB was not cleaved). Where the expression plasmid mediated the cleavage of the target plasmid (in the PC group, Cas9 was expressed to cleave ccdB; and in the DEAR1-DEAR3 groups, the corresponding intron RNA sequences were expressed respectively), the ccdB toxin gene cannot be expressed normally, allowing bacteria to survive on the ccdB induction expression plates; meanwhile, the bacteria died on the ampicillin plates due to loss of ampicillin resistance caused by cleavage of the target plasmid. The bacterial plate spreading results and the ccdB gene expression level analysis show that all of DEAR1-DEAR3 can mediate plasmid cleavage in *E. coli* cells.

### 5. Further verification of DEAR1-mediated plasmid interference in E. coli cells

Using primers GGATGAGTTTGCAAACAAAGTCCTTTCTGCCG (SEQ ID NO:45) and AGGACTTTGTTTGCAAACTCATCCAATGATACCTAGC (SEQ ID NO:46), the DEAR1 expression plasmid was subjected to PCR. A ΔTRS mutant expression plasmid was constructed via homologous recombination, designated as Dr1_ΔTRS (an expression plasmid with deletion of the 6-nucleotide TRS sequence in DEAR1), which served as one of the expression plasmids.

For the plasmid used in the NC group in Step 3, primers:
AGTACAGCATCGGCCTGGCCATCGGCACCAACTCTGTGG (SEQ ID NO:47); and GGCCAGGCCGATGCTGTACTTCTTGTCAGAACCGTGGTGA (SEQ ID NO:48) were used for PCR. The PCR product was further subjected to PCR using primers:
CCGACTACGATGTGGACGCCATCGTGCCTCAGAGCTTTC (SEQ ID NO:49) and GGCGTCCACATCGTAGTCGGACAGCCGGTTGATGTCC (SEQ ID NO:50). A dCas9 expression plasmid (with both enzymatic cleavage active site of Cas9 mutated and inactivated) was constructed via homologous recombination, designated as dCas9, which served as one of the expression plasmids.

For the plasmid used in the PC group in Step 3, primers:
CCGACTACGATGTGGACGCCATCGTGCCTCAGAGCTTTC (SEQ ID NO:49) and GGCGTCCACATCGTAGTCGGACAGCCGGTTGATGTCC (SEQ ID NO:50) were used for PCR. A nCas9 expression plasmid (with 1 enzymatic cleavage active site H840 of Cas9 mutated and inactivated) was constructed via homologous recombination, designated as nCas9, which served as one of the expression plasmids.

The plasmid used in the PC group in Step 3 was used without modification as the wtCas9 expression plasmid. The target plasmid constructed in Step 1 was co-transformed into the *E. coli* strain BW25141 (CGSC, Strain Accession No.: 7635) with the above different expression plasmids (dCas9, nCas9, wtCas9, Dr1_ΔTRS, and the DEAR1 expression plasmid used in Step 2), respectively. After culturing for a certain period, bacterial culture samples were taken and cultured on plates containing the antibiotic (ampicillin) against which the target plasmid was resistant. Referring to FIG. 13, when the bacteria contained only the target plasmid, they can survive on the ampicillin plates and colonies were formed (Blank group), which was essentially consistent with the survival/death situation of the bacterial colonies in which Dr1_ΔTRS (DEAR expression plasmid lacking the TRS) or dCas9 expression plasmid was transformed. The DEAR1 expression plasmid mediated cleavage of the target plasmid, which was essentially consistent with the results of nCas9 and wtCas9 expression plasmids; accordingly, the bacteria died on the ampicillin plates due to loss of ampicillin resistance caused by cleavage of the target plasmid. The bacterial plate spreading results and the AmpR gene expression level analysis show that DEAR1 can mediate plasmid cleavage as guided by the TRS region in *E. coli* cells.

### Example 5. Detection of plasmid interference by DEAR4-DEAR9 in E. coli cells

### 1. Construction of target plasmids

A ccdB toxin gene-inducible expression plasmid (Addgene, Plasmid ID: 69056) containing the corresponding intron RNA target sequences for DEAR1 and DEAR4-DEAR9 in the plasmid replication origin (ori) was used as the target plasmid.

### 2. Construction of other DEAR expression plasmids

The method was essentially the same as in Example 4. In the DEAR expression plasmids, the J23119 promoter (with the specific sequence of (SEQ ID NO: 42): TTGACAGCTAGCTCAGTCCTAGGTATAATACTAGT) was used to drive the expression of each DEAR sequence. The construction method was as follows: The J23119 promoter was ligated to each of Dr1_△TRS, DEAR1 and DEAR4-DEAR9 (where the Dr1_△TRS and DEAR1 were the same as those in Example 4). Then, the sequences of Dr1_△TRS, DEAR1 and DEAR4-DEAR9, each ligated to the J23119 promoter were individually inserted into the pCDFDuet1 plasmid vector (Novagen, Cat. No. 71340-3) via homologous recombination, thereby individually replacing the entire sequence of the 410-3765 region of this plasmid.

### 3. Detection of plasmid interference in E. coli cells

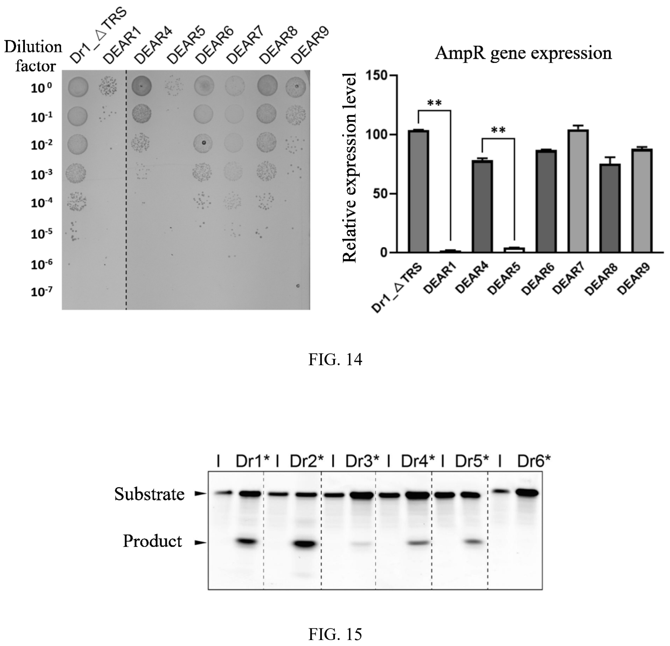

The method was essentially the same as in Example 4. The target plasmid in Step 1 was co-transformed into the *E. coli* strain BW25141 (CGSC, Strain Accession No.: 7635) with different expression plasmids (Dr1_△TRS, DEAR1 and DEAR4-DEAR9 expression plasmids) constructed in Step 2, respectively. After culturing for a certain period, bacterial culture samples were taken and cultured respectively on plates containing the antibiotic (ampicillin) against which the target plasmid was resistant. Referring to FIG. 14, the bacterial colonies survived when the transformed expression plasmid did not mediate the cleavage of the target plasmid (Dr1_△TRS, DEAR4, DEAR6, DEAR7, DEAR8 and DEAR9 groups expressing the corresponding intron RNA sequences respectively). The expression plasmid mediated cleavage of the target plasmid (DEAR1 and DEAR5 groups expressing the corresponding intron RNA sequences respectively); accordingly, the bacteria died on the ampicillin plates due to loss of ampicillin resistance caused by cleavage of the target plasmid. The bacterial plate spreading results and the Amp gene expression level analysis show that DEAR1 and DEAR5 can mediate plasmid cleavage in *E. coli* cells, while the DEAR4, DEAR6, DEAR7, DEAR8 and DEAR9 show no plasmid cleavage activity in *E. coli* cells.

### Example 6. In Vitro cleavage of plasmids by DEAR nucleic acid manipulation systems

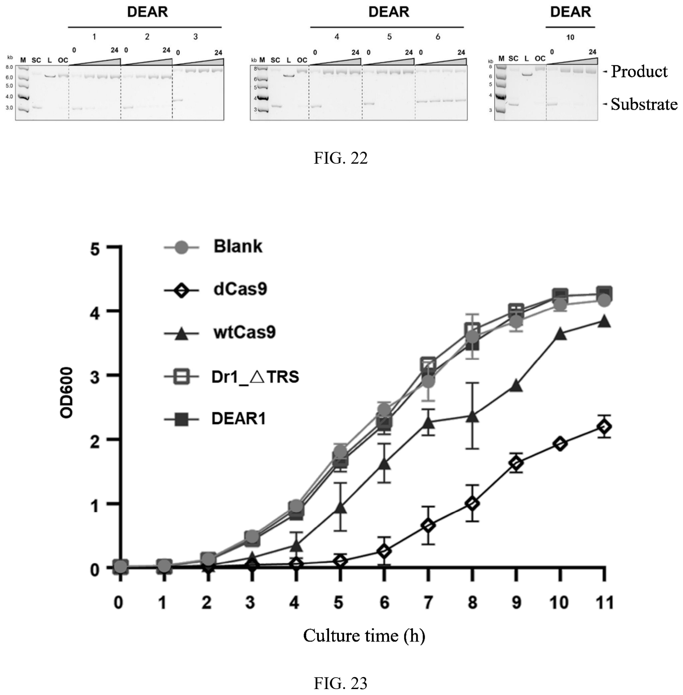

An inducible expression plasmid for the ccdB toxin gene (Addgene, Plasmid ID: 69056) containing the corresponding target sequences for DEAR1-DEAR6 and DEAR10 was used as the plasmid substrate for the *in vitro* cleavage experiment. DEAR1-DEAR6 and DEAR10 (1.5 µM) were individually incubated with the plasmid substrate (0.03 µM) under conditions of 150 mM KCl, 10 mM MgCl₂, 40 mM MOPS 7.5, and 37°C. Samples were taken at given time points (0 h, 3 h, 8 h, and 24 h). After the reaction was terminated, agarose gel electrophoresis was performed, and the gel image was obtained by taking a photograph on an ultraviolet imager. The results are shown in FIG. 22, in which L represents the plasmid treated with EcoRI (NEB, Cat. No. R0101V) in a linear double-stranded state; OC represents the plasmid treated with Nt.BspQI (NEB, Cat. No. R0644S) in an open circular state; SC represents the untreated plasmid in a supercoiled state; and 0, 3, 8, and 24 represent the time for the plasmid to be cleaved with DEAR (in hours). Referring to FIG. 22, the substrate plasmids exist in a supercoiled state, and the products resulting from the cleavage reaction appear in an open circular state and are located above the substrates. It can be seen that all DEAR1-DEAR6 and DEAR10 can cleave the plasmid.

### Example 7. Toxicity test of DEAR nucleic acid manipulation system in E. coli

In order to eliminate the influence of the DEAR nucleic acid manipulation system itself on the growth rate of *E. coli,* turbidimetry was used to determine the growth curve of *E. coli* in this Example. 50 ng of each of the Blank, dCas9, Cas9, Dr1_△TRS, and DEAR1 expression plasmids (as in Example 4) was individually taken and chemically transformed into *E. coli* BW25141 competent cells. Plasmid was added to the competent cell suspension, and the mixture was mixed evenly and incubated for 30 min on ice; and placed in a water bath at 42°C for 60 s, and in an ice bath for 2 min. 1 mL of liquid LB medium was added, and the mixture was cultured for resuscitation at 37°C in a constant temperature shaker at 220 rpm for 1 h. After resuscitation, the cells were plated on LB plates containing streptomycin (50 ng/mL) and cultured invertedly at 37°C in a constant temperature incubator for 16 h. Single colonies were picked from the plates and transferred to 1 mL of liquid LB medium, and the mixture was cultured at 37°C in a constant temperature shaker at 220 rpm for about 3-6 h. 2 µL of bacterial culture was aspirated each time, and the OD600 value was measured using a Nanodrop One microvolume spectrophotometer until it reached 0.5-0.6. 1 OD of bacterial culture (OD600 = 0.6) was aspirated and transferred to 100 mL of liquid LB medium, and the mixture was cultured at 37°C in a constant temperature shaker at 220 rpm. At 0, 1, 2, 3, 4, 5, 6, 7, 8, 9, 10, and 11 h of culture, 2 µL of bacterial culture was aspirated, and the OD600 value was measured using a Nanodrop One microvolume spectrophotometer. Using the time point as the abscissa and the OD600 value as the ordinate, the bacterial growth curve was plotted using GraphPad 6.0. As shown in FIG. 23, the expression of Dr1_△TRS and DEAR1 has little influence on the growth of *E. col.*

### Example 8. Cleavage of new DNA sites by DEARs with reprogrammed TRS

Single-stranded DNA substrate with a new DEAR target sequence (in which the underlined and bolded part is the target sequence recognized by DEAR) was synthesized according to the sequence shown in Table 5 below, and was labeled with -Cy5 at the 3' end. DEAR1-DEAR6 with the TRS sequence altered to CGAUAG (1.5 µM) were each incubated with this single-stranded DNA substrate (100 nM) under conditions of 50 mM MgCl₂, 10 mM KC1, 40 mM MOPS 7.5, and 37°C for 8 h. After the reaction was terminated, Urea-PAGE electrophoresis was performed, and the gel fluorescence signals were scanned on a fluorescence imager. The results are shown in FIG. 15. As shown in FIG. 15, the cleavage products of ssDNA are located below the substrate, and it can be seen that all DEAR1-DEAR6 can cleave the new single-stranded DNA. In FIG. 15, I represents the input ssDNA substrate, and Dr1*-Dr6* represent the corresponding cleavage products of ssDNA by DEAR1-DEAR6 with the TRS altered.

**Table 5:**

| ID # | SEQ ID NO: | Sequences (5'-3'; in which the underlined and bolded part is the target sequence) |
|---|---|---|
| Re-DEAR | SEQ ID NO: 51 | ACCAAA**CTATCG**AGCGGATCGGAT |

DEAR10 with a reprogrammed TRS (1.5 µM), with the TRS sequence altered to CGAUAG in DEAR10, was incubated with the single-stranded DNA substrate with the corresponding new target sequence (100 nM, SEQ ID NO: 51) under conditions of 50 mM MgCl₂, 10 mM KCl, 40 mM MOPS 7.5, and 37°C for 8 h. After the reaction was terminated, Urea-PAGE electrophoresis was performed, and the gel fluorescence signals were scanned on a fluorescence imager. The results are shown in FIG. 21. As shown in FIG. 21, the cleavage product of ssDNA is located below the substrate, and it can be seen that DEAR10 with a reprogrammed TRS can cleave the new single-stranded DNA. In FIG. 21, I represents the input ssDNA substrate, and Dr10* represents the cleavage product of ssDNA by DEAR10 with the TRS altered.

### Example 9. Genomic DNA cleavage in mammalian cells-1

### 1. Construction of stable transfection plasmids

The DEAR1 target sequence stable transfection plasmid and the DEAR stable transfection plasmid were constructed using the PiggyBac^{™} Transposon Vector System (from System Biosciences).
(1) Construction of DEAR1 target sequence stable transfection plasmid: The sequence of the frameshifted puromycin resistance (PuroR) gene with the DEAR1 target sequence at N-terminus
   (GCTAGCGCCACCATGTCCGGTAGCGGTGGCTCAAGCGGAAGC**TGTCTTAAGACA**TTCTTGCGGAA GTGGGTCTGGCTCAGGAGG**TGTCTTAAGACA**GTCCGGTTCAAGTGGAAGTTCAAGTGGAAG**TGTCTTAAG ACA**TTCTTGTGGAGGTTCCTCTGGTAGTaccgagtacaagcccacggtgcgcctcgccacccgcgacgacgtccccagggccgtacgcaccctcgc cgccgcgttcgccgactaccccgccacgcgccacaccgtcgatccggaccgccacatcgagcgggtcaccgagctgcaagaactcttcctcacgcgcgtcgggctcgacatc ggcaaggtgtgggtcgcggacgacggcgccgcggtggcggtctggaccacgccggagagcgtcgaagcgggggcggtgttcgccgagatcggcccgcgcatggccgagt tgagcggttcccggctggccgcgcagcaacagatggaaggcctcctggcgccgcaccggcccaaggagcccgcgtggttcctggccaccgtcggagtctcgcccgaccacc agggcaagggtctgggcagcgccgtcgtgctccccggagtggaggcggccgagcgcgccggggtgcccgccttcctggagacctccgcgccccgcaacctccccttctacg agcggctcggcttcaccgtcaccgccgacgtcgaggtgcccgaaggaccgcgcacctggtgcatgacccgcaagcccggtgcctgataa (SEQ ID NO: 52, in which the uppercase letters are the DEAR1 target sequence; the bolded and underlined letters are the sites that DEAR1 can specifically recognize and cleave, the sense and antisense strands of which can be cleaved by DEAR1, resulting in a double strand break; and the lowercase letters are the PuroR gene)) was inserted at the XbaI cleavage site within the multiple cloning site of the PiggyBac Dual promoter PB513B-1 plasmid via homologous recombination, and the blasticidin resistance (Blasticidine S-deaminase) gene
   (atggccaagcctttgtctcaagaagaatccaccctcattgaaagagcaacggctacaatcaacagcatccccatctctgaagactacagcgtcgccagcgcagctc tctctagcgacggccgcatcttcactggtgtcaatgtatatcattttactgggggaccttgtgcagaactcgtggtgctgggcactgctgctgctgcggcagctggcaacctgacttgt atcgtcgcgatcggaaatgagaacaggggcatcttgagcccctgcggacggtgccgacaggtgcttctcgatctgcatcctgggatcaaagccatagtgaaggacagtgatgga cagccgacggcagttgggattcgtgaattgctgccctctggttatgtgtgggagggctaa (SEQ ID NO: 53)) was inserted between the NcoI and SalI cleavage sites via homologous recombination.
(2) Construction of DEAR stable transfection plasmid: The DEAR1 sequence driven by the U6 promoter and terminated with the TTTTTTTT signal
   **(AGACTAGACGGTTCCTGGCCTTTTGCTGGCCTTTTGCTCACATGTGAGGGCCTATTTCCCAT GATTCCTTCATATTTGCATATACGATACAAGGCTGTTAGAGAGATAATTGGAATTAATTTGACTGTAAA CACAAAGATATTAGTACAAAATACGTGACGTAGAAAGTAATAATTTCTTGGGTAGTTTGCAGTTTTAA AATTATGTTTTAAAATGGACTATCATATGCTTACCGTAACTTGAAAGTATTTCGATTTCTTGGCTTTAT ATATCTTGTGGAAAGGACGAAACACC**GTGCGCTCGGCATGGGTGCAATCTCTAGGGTGAAAGTCCCGAAC TGCGAAGGCAGAAGTAGCAGTTAGCTTAACGCAAGGGTGTCCGTGGTGACGCGGAATCTGAAGGAAGCGG GCGGCAAACTTCCGGTCTGAGGAACACGAACTTCATATAAGGCTAGGTATCATTGGATGAGTTTGCAAGAC AAAACAAAGTCCTTTCTGCCGAAGGTGATACAGAGTAAATGAAGCAGATAGATGGAAGGAAAGATTGTACT CTTACCCGAGGAGGTCTGATGGATACGTGAAGTGCGCTTCATAACCTACTTAGTGATAAGTAACTGAACCAT CAGAAGTCAGCAGAGGTCATAGTACGAATCGGTCTAGAACGATTCGGAAGGACTGAACAATCAAGAGAAA ATAGCCCTTGGCATTCAGTACGTCATGATGAACACAGAAAACATGGTACCTCCCAAGAGAAAGGAAACGGT GAATCCCGTGGGAATCTTTTGGAGGGTGGAGTGACGACTGGCATAAGAAGATCAGCTATTTACGGAAGGAA GCTTGCGTCATTATCTTGATTGAACCGCCGTATACGGAACCGTACGTACGGTGGTGTGAGAGGACGGAGGTT AATCACCTCCTCCTACTCGATttttttttggtaccgacattgattattgactagtcatgtctg (SEQ ID NO: 54, in which the uppercase, bolded and underlined letters are the U6 promoter sequence; the uppercase, unbolded and non-underlined letters are the corresponding DNA sequence of DEAR1; and the lowercase letters are the transcription termination signal and partial vector backbone sequence)), or the DEAR-NT sequence driven by the U6 promoter and terminated with the TTTTTTTT signal
   **(AGACTAGACGGTTCCTGGCCTTTTGCTGGCCTTTTGCTCACATGTGAGGGCCTATTTCCCAT GATTCCTTCATATTTGCATATACGATACAAGGCTGTTAGAGAGATAATTGGAATTAATTTGACTGTAAA CACAAAGATATTAGTACAAAATACGTGACGTAGAAAGTAATAATTTCTTGGGTAGTTTGCAGTTTTAA AATTATGTTTTAAAATGGACTATCATATGCTTACCGTAACTTGAAAGTATTTCGATTTCTTGGCTTTAT ATATCTTGTGGAAAGGACGAAACACC**GTACGACGGGCATGTCGTACATCTGAGGTGCAAGTCCTCTGTGG ACACCTGCTACCGGTGAACCCAATAGCGACTCCCAAGCCTGACTATCGCGAGGTAACGGGGAGAGGAAGGG ATAGCGAAATCGTGGCTCTACGAACAGAAACGTGATATTAAGGCGTATATAGTGGATGAGGGGGCTAGGCA CTCTAAAGTCCAAAAAGGTAGTCGTAATCTATGTGCGTAAATCACGAGAGTAAACGAGGAAAGATGTACGA CTTATCCCGTGAGGTCTCATGAGCGTCAAGTAGACGTAGTAATAACGAATCATGAGAAGTCAGCCGAGGTC ATAGTAGTGATGAAGTTTCTGTAATGGAAACGGAGCGAAGGACCGAACGATTAAATGATACCCTATCTAAC TGATTTTGTCTGCCCTAACACCTGGTCTGAAATGACGGTACTCGCAGAACGTATGCTGAAAGAGGCTGCTCA CAAATATAGGACGGCACAAGCGGAACGAAATCATATATAGGTAGGTGTGGGAACAAAAAAAGTGTTCTCGC CAAAGTTTAGTTGAACCGCCGTGTGCCGAACGGCACGCACGGTGGTGTGAGAGGGACTATCAATTAGTCCC TACTCGATttttttttggtaccgacattgattattgactagtcatgtctg (SEQ ID NO: 55, in which the uppercase, bolded and underlined letters are the U6 promoter sequence; the uppercase, unbolded and non-underlined letters are the DEAR-NT sequence (the corresponding DNA sequence of DEAR2); and the lowercase letters are the transcription termination signal and partial vector backbone sequence)) was individually inserted between the SfiI and MluI cleavage sites in the PiggyBac Dual promoter PB513B-1 plasmid via homologous recombination, and the hygromycin resistance (HygBR) gene
   (atgggtaaaaagcctgaactcaccgcgacgtctgtcgagaagtttctgatcgaaaagttcgacagcgtttccgacctgatgcagctctcggagggcgaagaatctc gtgctttcagcttcgatgtaggagggcgtggatatgtcctgcgggtaaatagctgcgccgatggtttctacaaagatcgttatgtttatcggcactttgcatcggccgcgctcccgatt ccggaagtgcttgacattggggaattcagcgagagcctgacctattgcatctcccgccgtgcacagggtgtcacgttgcaagacctgcctgaaaccgaactgcccgctgttctgca gccggtcgcggaggcaatggatgcgatcgctgcggccgatcttagccagacgagcgggttcggcccattcggaccgcaaggaatcggtcaatacactacatggcgtgatttcat atgcgcgattgctgatccccatgtgtatcactggcaaactgtgatggacgacaccgtcagtgcgtccgtcgcgcaggctctcgatgagctgatgctttgggccgaggactgccccg aagtccggcacctcgtgcacgcggatttcggctccaacaatgtcctgacggacaatggccgcataacagcggtcattgactggagcgaggcgatgttcggggattcccaatacg aggtcgccaacatcttcttctggaggccgtggttggcttgtatggagcagcagacgcgctacttcgagcggaggcatccggagcttgcaggatcgccgcggctccgggcgtatat gctccgcattggtcttgaccaactctatcagagcttggttgacggcaatttcgatgatgcagcttgggcgcagggtcgatgcgacgcaatcgtccgatccggagccgggactgtcg ggcgtacacaaatcgcccgcagaagcgcggccgtctggaccgatggctgtgtagaagtactcgccgatagtggaaaccgacgccccagcactcgtccgagggcaaaggaata a (SEQ ID NO: 26)) was individually inserted between the NcoI and SalI cleavage sites via homologous recombination to individually obtain the DEAR1 stable transfection plasmid and the DEAR-NT stable transfection plasmid.

### 2. Stable transfection with DEAR target sequence plasmid and DEAR plasmid and resistance screening-mediated enrichment

HEK-293T cells (ATCC CRL-11268) were cultured in high glucose DMEM medium containing 10% fetal bovine serum at 37°C with 5% CO₂ until they reached the logarithmic phase. The cells were digested with 0.25% trypsin, then washed twice with PBS (pH 7.0-7.2), and resuspended in Opti-MEM^{™} medium (Gibco, Cat. No. 31985070), with the cell density adjusted to 5 × 10⁴ cells/µL. 2 µg of Integration PB transposase plasmid (System Biosciences) and 2 µg of DEAR1 target sequence stable transfection plasmid were added to 20 µL of cell suspension, and the mixed cell suspension was subjected to electroporation at 450 V (Celetrix Biotechnologies, model: LE+). The electroporated cells were added to high glucose DMEM medium containing 10% fetal bovine serum. 24 h post-electroporation, the medium was replaced with a medium containing 10 µg/mL of blasticidin. Antibiotic resistance screening was performed for one week, during which the cells were passaged based on their growth status. Once the cell state had stabilized, a stable transfection cell line containing the DEAR1 target sequence was obtained. In the same way, the stable transfection cell line containing the DEAR1 target sequence was electroporated with 2 µg of Integration PB transposase plasmid (System Biosciences) and 2 µg of DEAR stable transfection plasmid (the DEAR1 stable transfection plasmid or DEAR-NT stable transfection plasmid). 24 h post-electroporation, the medium was replaced with a medium containing 50 µg/mL of hygromycin B. Antibiotic resistance screening was performed for one week, during which the cells were passaged based on their growth status. Once the cell state had stabilized, the medium was replaced with a medium containing 10 µg/mL of puromycin. Antibiotic resistance screening was performed for one week.

For the stable transfection cell line containing the DEAR1 target sequence, it lacked resistance to puromycin since the PuroR gene integrated in the cells was frameshifted and cannot express the functional protein. DEAR1 (the DEAR1 stable transfection plasmid) can cleave the DEAR1 target sequence to induce DNA double-strand breaks, and the insertion or deletion mutations introduced during the repair of these breaks can restore the frameshifted PuroR gene to allow it to express normally, resulting in cell survival under puromycin screening. In contrast, DEAR-NT (the DEAR-NT stable transfection plasmid) cannot cleave the DEAR1 target sequence, and the cells cannot express the functional PuroR gene, resulting in cell death under puromycin screening. As shown in FIG. 16, the cells stably transfected with DEAR1 (the DEAR1 stable transfection plasmid) survived, while the cells stably transfected with DEAR-NT (the DEAR-NT stable transfection plasmid) died.

### 3. Verification of cleavage of genomic DNAs in mammalian cells by DEAR1 through next-generation sequencing

For the surviving cells (stably transfected with DEAR1, i.e., the DEAR1 stable transfection plasmid) in FIG. 16, the genome was extracted, and a next-generation sequencing library for the DEAR1 target sequence was constructed using the TIANSeq Fast DNA Library Kit (Illumina). Next-generation sequencing was performed by Novogene. The next-generation sequencing data was analyzed online using the CRISPResso2 website. The results are shown in FIG. 17A, in which 47.14% of reads were mutated. FIG. 17B shows a sequence alignment plot of reads near the first and second cleavage sites within the DEAR1 target sequence, in which both insertion and deletion mutations occurred near the DEAR1 cleavage site (the position indicated by the dotted line in the figure). The sequencing data indicate that DEAR1 has the activity of specifically cleaving genomic double-stranded DNA in mammalian cells.

### Example 10. Genomic DNA cleavage in mammalian cells-2

### 1. Construction of stable transfection plasmids

The DEAR1 target sequence stable transfection plasmid and the DEAR stable transfection plasmid were constructed using the PiggyBac^{™} Transposon Vector System (from System Biosciences).
(1) Construction of DEAR1 target sequence stable transfection plasmid: The sequence of the frameshifted puromycin resistance (PuroR) gene with the DEAR1 target sequence at N-terminus
   (GCTAGCGCCACCATGTCCGGTAGCGGTGGCTCAAGCGGAAGC**TGTCTTAAGACA**TTCTTGCGGAA GTGGGTCTGGCTCAGGAGG**TGTCTTAAGACA**GTCCGGTTCAAGTGGAAGTTCAAGTGGAAG**TGTCTTAAG ACA**TTCTTGTGGAGGTTCCTCTGGTAGTaccgagtacaagcccacggtgcgcctcgccacccgcgacgacgtccccagggccgtacgcaccctcgc cgccgcgttcgccgactaccccgccacgcgccacaccgtcgatccggaccgccacatcgagcgggtcaccgagctgcaagaactcttcctcacgcgcgtcgggctcgacatc ggcaaggtgtgggtcgcggacgacggcgccgcggtggcggtctggaccacgccggagagcgtcgaagcgggggcggtgttcgccgagatcggcccgcgcatggccgagt tgagcggttcccggctggccgcgcagcaacagatggaaggcctcctggcgccgcaccggcccaaggagcccgcgtggttcctggccaccgtcggagtctcgcccgaccacc agggcaagggtctgggcagcgccgtcgtgctccccggagtggaggcggccgagcgcgccggggtgcccgccttcctggagacctccgcgccccgcaacctccccttctacg agcggctcggcttcaccgtcaccgccgacgtcgaggtgcccgaaggaccgcgcacctggtgcatgacccgcaagcccggtgcctgataa (SEQ ID NO: 52, in which the uppercase letters are the DEAR1 target sequence; the bolded and underlined letters are the target sites that DEAR1 can specifically recognize and cleave, the sense and antisense strands of which 3 sites (Target 1-Target 3) can be cleaved by DEAR1, resulting in a double strand break; and the lowercase letters are the PuroR gene, in which sequence PuroR is 148 bp apart from the translation start site ATG and is therefore in a frameshifted state)) was inserted at the position of the XbaI cleavage site into the multiple cloning site of the PiggyBac Dual promoter PB513B-1 plasmid via homologous recombination, and the blasticidin resistance (Blasticidine S-deaminase) gene
   (atggccaagcctttgtctcaagaagaatccaccctcattgaaagagcaacggctacaatcaacagcatccccatctctgaagactacagcgtcgccagcgcagctc tctctagcgacggccgcatcttcactggtgtcaatgtatatcattttactgggggaccttgtgcagaactcgtggtgctgggcactgctgctgctgcggcagctggcaacctgacttgt atcgtcgcgatcggaaatgagaacaggggcatcttgagcccctgcggacggtgccgacaggtgcttctcgatctgcatcctgggatcaaagccatagtgaaggacagtgatgga cagccgacggcagttgggattcgtgaattgctgccctctggttatgtgtgggagggctaa (SEQ ID NO: 53)) was inserted between the NcoI and SalI cleavage sites via homologous recombination.
(2) Construction of DEAR stable transfection plasmid: The DEAR1 sequence driven by the U6 promoter and terminated with the TTTTTTTT signal
   **(AGACTAGACGGTTCCTGGCCTTTTGCTGGCCTTTTGCTCACATGTGAGGGCCTATTTCCCAT GATTCCTTCATATTTGCATATACGATACAAGGCTGTTAGAGAGATAATTGGAATTAATTTGACTGTAAA CACAAAGATATTAGTACAAAATACGTGACGTAGAAAGTAATAATTTCTTGGGTAGTTTGCAGTTTTAA AATTATGTTTTAAAATGGACTATCATATGCTTACCGTAACTTGAAAGTATTTCGATTTCTTGGCTTTAT ATATCTTGTGGAAAGGACGAAACACC**GTGCGCTCGGCATGGGTGCAATCTCTAGGGTGAAAGTCCCGAAC TGCGAAGGCAGAAGTAGCAGTTAGCTTAACGCAAGGGTGTCCGTGGTGACGCGGAATCTGAAGGAAGCGG GCGGCAAACTTCCGGTCTGAGGAACACGAACTTCATATAAGGCTAGGTATCATTGGATGAGTTTGCAAGAC AAAACAAAGTCCTTTCTGCCGAAGGTGATACAGAGTAAATGAAGCAGATAGATGGAAGGAAAGATTGTACT CTTACCCGAGGAGGTCTGATGGATACGTGAAGTGCGCTTCATAACCTACTTAGTGATAAGTAACTGAACCAT CAGAAGTCAGCAGAGGTCATAGTACGAATCGGTCTAGAACGATTCGGAAGGACTGAACAATCAAGAGAAA ATAGCCCTTGGCATTCAGTACGTCATGATGAACACAGAAAACATGGTACCTCCCAAGAGAAAGGAAACGGT GAATCCCGTGGGAATCTTTTGGAGGGTGGAGTGACGACTGGCATAAGAAGATCAGCTATTTACGGAAGGAA GCTTGCGTCATTATCTTGATTGAACCGCCGTATACGGAACCGTACGTACGGTGGTGTGAGAGGACGGAGGTT AATCACCTCCTCCTACTCGATttttttttggtaccgacattgattattgactagtcatgtctg (SEQ ID NO: 54, in which the uppercase, bolded and underlined letters are the U6 promoter sequence; the uppercase, unbolded and non-underlined letters are the corresponding DNA sequence of DEAR1; and the lowercase letters are the transcription termination signal and partial vector backbone sequence)), or the DEAR1-NT sequence driven by the U6 promoter and terminated with the TTTTTTTT signal
   **(****AGACTAGACGGTTCCTGGCCTTTTGCTGGCCTTTTGCTCACATGTGAGGGCCTATTTCCCAT GATTCCTTCATATTTGCATATACGATACAAGGCTGTTAGAGAGATAATTGGAATTAATTTGACTGTAAA CACAAAGATATTAGTACAAAATACGTGACGTAGAAAGTAATAATTTCTTGGGTAGTTTGCAGTTTTAA AATTATGTTTTAAAATGGACTATCATATGCTTACCGTAACTTGAAAGTATTTCGATTTCTTGGCTTTAT ATATCTTGTGGAAAGGACGAAACACC**GTGCGCTCGGCATGGGTGCAATCTCTAGGGTGAAAGTCCCGAAC TGCGAAGGCAGAAGTAGCAGTTAGCTTAACGCAAGGGTGTCCGTGGTGACGCGGAATCTGAAGGAAGCGG GCGGCAAACTTCCGGTCTGAGGAACACGAACTTCATATAAGGCTAGGTATCATTGGATGAGTTTGCTAAGGT AAACAAAGTCCTTTCTGCCGAAGGTGATACAGAGTAAATGAAGCAGATAGATGGAAGGAAAGATTGTACTC TTACCCGAGGAGGTCTGATGGATACGTGAAGTGCGCTTCATAACCTACTTAGTGATAAGTAACTGAACCATC AGAAGTCAGCAGAGGTCATAGTACGAATCGGTCTAGAACGATTCGGAAGGACTGAACAATCAAGAGAAAA TAGCCCTTGGCATTCAGTACGTCATGATGAACACAGAAAACATGGTACCTCCCAAGAGAAAGGAAACGGTG AATCCCGTGGGAATCTTTTGGAGGGTGGAGTGACGACTGGCATAAGAAGATCAGCTATTTACGGAAGGAAG CTTGCGTCATTATCTTGATTGAACCGCCGTATACGGAACCGTACGTACGGTGGTGTGAGAGGACGGAGGTTA ATCACCTCCTCCTACTCGATttttttttggtaccgacattgattattgactagtcatgtctg (SEQ ID NO: 58, in which the uppercase, bolded and underlined letters are the U6 promoter sequence; the uppercase, unbolded and non-underlined letters are the DEAR1-NT sequence (the non-target control, that is, the TRS sequence of DEAR1 is altered to the italicized TAAGGT, thereby preventing it from targeting and cleaving the original DEAR1 target sequence); and the lowercase letters are the transcription termination signal and partial vector backbone sequence)) was individually inserted between the SfiI and MluI cleavage sites in the PiggyBac Dual promoter PB513B-1 plasmid via homologous recombination, and the hygromycin resistance (HygBR) gene
   (atgggtaaaaagcctgaactcaccgcgacgtctgtcgagaagtttctgatcgaaaagttcgacagcgtttccgacctgatgcagctctcggagggcgaagaatctc gtgctttcagcttcgatgtaggagggcgtggatatgtcctgcgggtaaatagctgcgccgatggtttctacaaagatcgttatgtttatcggcactttgcatcggccgcgctcccgatt ccggaagtgcttgacattggggaattcagcgagagcctgacctattgcatctcccgccgtgcacagggtgtcacgttgcaagacctgcctgaaaccgaactgcccgctgttctgca gccggtcgcggaggcaatggatgcgatcgctgcggccgatcttagccagacgagcgggttcggcccattcggaccgcaaggaatcggtcaatacactacatggcgtgatttcat atgcgcgattgctgatccccatgtgtatcactggcaaactgtgatggacgacaccgtcagtgcgtccgtcgcgcaggctctcgatgagctgatgctttgggccgaggactgccccg aagtccggcacctcgtgcacgcggatttcggctccaacaatgtcctgacggacaatggccgcataacagcggtcattgactggagcgaggcgatgttcggggattcccaatacg aggtcgccaacatcttcttctggaggccgtggttggcttgtatggagcagcagacgcgctacttcgagcggaggcatccggagcttgcaggatcgccgcggctccgggcgtatat gctccgcattggtcttgaccaactctatcagagcttggttgacggcaatttcgatgatgcagcttgggcgcagggtcgatgcgacgcaatcgtccgatccggagccgggactgtcg ggcgtacacaaatcgcccgcagaagcgcggccgtctggaccgatggctgtgtagaagtactcgccgatagtggaaaccgacgccccagcactcgtccgagggcaaaggaata a (SEQ ID NO: 26)) was individually inserted between the NcoI and SalI cleavage sites via homologous recombination to individually obtain the DEAR1 stable transfection plasmid and the DEAR1-NT stable transfection plasmid.

### 2. Stable transfection with DEAR target sequence plasmid and DEAR plasmid and resistance screening-mediated enrichment

HEK-293T cells (ATCC CRL-11268) were cultured in high glucose DMEM medium containing 10% fetal bovine serum at 37°C with 5% CO₂ until they reached the logarithmic phase. The cells were digested with 0.25% trypsin, then washed twice with PBS (pH 7.0-7.2), and resuspended in Opti-MEM^{™} medium (Gibco, Cat. No. 31985070), with the cell density adjusted to 5 × 10⁴ cells/µL. 2 µg of Integration PB transposase plasmid (System Biosciences) and 2 µg of DEAR1 target sequence stable transfection plasmid were added to 20 µL of cell suspension, and the mixed cell suspension was subjected to electroporation at 450 V (Celetrix Biotechnologies, model: LE+). The electroporated cells were added to high glucose DMEM medium containing 10% fetal bovine serum. 24 h post-electroporation, the medium was replaced with a medium containing 10 µg/mL of blasticidin. Antibiotic resistance screening was performed for one week, during which the cells were passaged based on their growth status. Once the cell state had stabilized, a stable transfection cell line containing the DEAR1 target sequence was obtained. In the same way, the stable transfection cell line containing the DEAR1 target sequence was electroporated with 2 µg of Integration PB transposase plasmid (System Biosciences) and 2 µg of DEAR stable transfection plasmid (the DEAR1 stable transfection plasmid or DEAR1-NT stable transfection plasmid). 24 h post-electroporation, the medium was replaced with a medium containing 50 µg/mL of hygromycin B. Antibiotic resistance screening was performed for one week, during which the cells were passaged based on their growth status. Once the cell state had stabilized, the medium was replaced with a medium containing 10 µg/mL of puromycin. Antibiotic resistance screening was performed for one week.

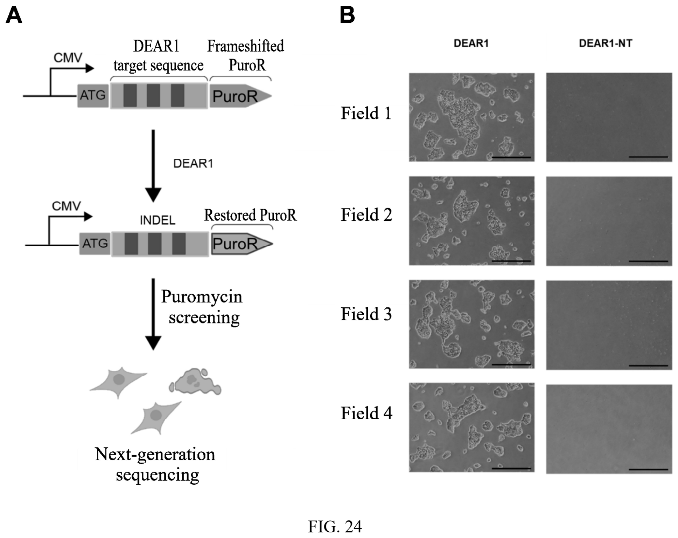

For the stable transfection cell line containing the DEAR1 target sequence, it lacked resistance to puromycin since the PuroR gene integrated in the cells was frameshifted and cannot express the functional protein. DEAR1 (the DEAR1 stable transfection plasmid) can cleave the DEAR1 target sequence to induce DNA double-strand breaks, and the insertion or deletion mutations (INDELs) introduced during the repair of these breaks can restore the frameshifted PuroR gene to allow it to express normally, resulting in cell survival under puromycin screening, as shown in FIG. 24A. In contrast, DEAR1-NT (the DEAR1-NT stable transfection plasmid) cannot cleave the DEAR1 target sequence, and the cells cannot express the functional PuroR gene, resulting in cell death under puromycin screening. As shown in FIG. 24B, the cells stably transfected with DEAR1 (the DEAR1 stable transfection plasmid) survived, while the cells stably transfected with DEAR1-NT (the DEAR1-NT stable transfection plasmid) died (scale bar: 500 µm).

### 3. Verification of cleavage of genomic DNAs in mammalian cells by DEAR1 through next-generation sequencing

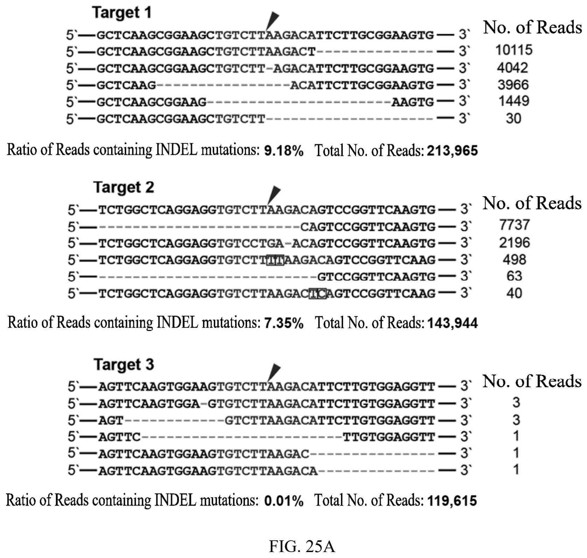

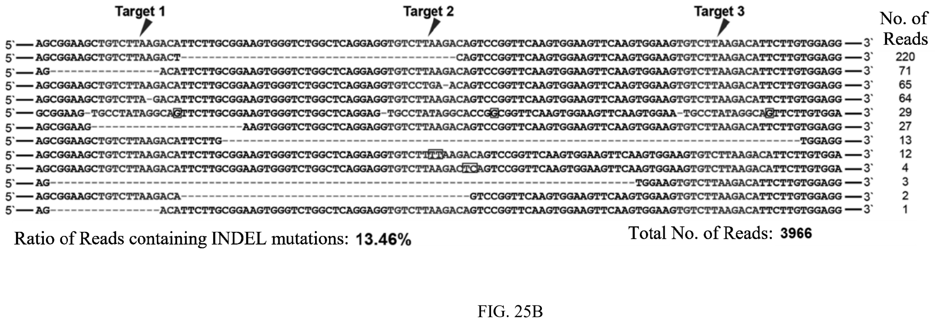

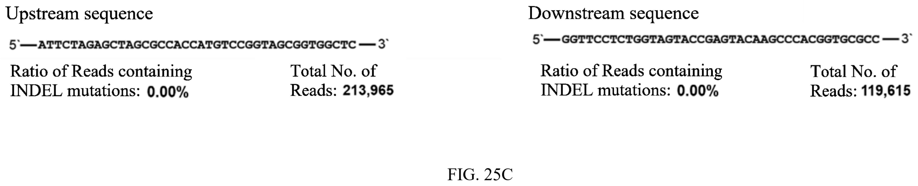

For the surviving cells (stably transfected with DEAR1, i.e., the DEAR1 stable transfection plasmid) in FIG. 24B, the genome was extracted, and a next-generation sequencing library for the DEAR1 target sequence was constructed using the TIANSeq Fast DNA Library Kit (Illumina). Next-generation sequencing was performed by Novogene. The next-generation sequencing data was analyzed online using the CRISPResso2 website. Mutations at the three target sites (Target 1-Target 3) within the DEAR1 target sequence were individually analyzed. The results are shown in FIG. 25A, in which the arrow indicates the cleavage site for DEAR1, the short bar indicates the deletion mutation, and the box indicates the insertion mutation. In Target 1, 9.18% of insertion or deletion mutations were detected; in Target 2, 7.35% of insertion or deletion mutations were detected; and in Target 3, 0.01% of insertion or deletion mutations were detected. Specifically, insertion mutations of between 1 to 2 nt in length and deletion mutations of between 1 to 25 nt in length near the three target sites were detected in this Example. In addition, by analyzing the full-length DEAR1 target sequence as a whole, as shown in FIG. 25B, deletion mutations spanning Target 1 and Target 2 with a maximum length of 85 nt were also observed in this Example. Analysis of the sequences upstream and downstream of the DEAR1 target sequence showed that, as shown in FIG. 25C, no insertion or deletion mutations were detected upstream and downstream of the DEAR1 target sequence, indicating that cleavage of genomic DNA in mammalian cells by DEAR1 is specifically guided by the TRS.

## Claims

1. A DEAR nucleic acid manipulation system, wherein the DEAR nucleic acid manipulation system comprises an RNA molecule derived from a bacterial Group IIC intron, and the RNA molecule comprises a target recognition site that hybridizes to a target sequence in a target nucleic acid.

2. The DEAR nucleic acid manipulation system of claim 1, wherein the DEAR nucleic acid manipulation system comprises at least one of Domain I, Domain II, Domain III, Domain IV, Domain V, and Domain VI; and
preferably, the DEAR nucleic acid manipulation system comprises at least Domain I, Domain II, Domain III, and Domain V.

3. The DEAR nucleic acid manipulation system of claim 1 or 2, wherein the DEAR nucleic acid manipulation system is in the range of 100-5660 nt, preferably 124-3897 nt, in length.

4. The DEAR nucleic acid manipulation system of any of claims 1-3, wherein the Domain I comprises 2-6 stem-loop/hairpin structures in the range of 50-400 nt in length; and preferably, the Domain I comprises 3-5 stem-loop/hairpin structures in the range of 65-384 nt in length; and/or,
the Domain II comprises 1-4 stem-loop/hairpin structures in the range of 10-300 nt in length; and preferably, the Domain II comprises 1-3 stem-loop/hairpin structures in the range of 10-218 nt in length; and/or,
the Domain III comprises 1-3 stem-loop/hairpin structures in the range of 10-200 nt in length; and preferably, the Domain III comprises 1-2 stem-loop/hairpin structures in the range of 10-140 nt in length; and/or,
the Domain IV comprises 0-4 stem-loop/hairpin structures in the range of 0-4500 nt in length; and preferably, the Domain IV comprises 0-4 stem-loop/hairpin structures in the range of 0-3000 nt in length; and/or,
the Domain V comprises 1 stem-loop/hairpin structure in the range of 20-60 nt in length; and preferably, the Domain V comprises 1 stem-loop/hairpin structure in the range of 29-43 nt in length; and/or,
the Domain VI comprises 1 stem-loop/hairpin structure in the range of 10-200 nt in length; and preferably, the Domain VI comprises 1 stem-loop/hairpin structure in the range of 10-112 nt in length.

5. The DEAR nucleic acid manipulation system of any of claims 1-4, wherein the Group IIC intron is a Group IIC intron in which an open reading frame encoding an intron-encoded protein is present or absent in Domain IV; optionally, the open reading frame encoding an intron-encoded protein is 0-4000 nt in length; and/or,
the target recognition site is located in the Domain I.

6. The DEAR nucleic acid manipulation system of any of claims 1-5, wherein the nucleotide sequence of the RNA molecule is selected from any one of:
(i) the nucleotide sequence comprises a nucleotide sequence as set forth in any one of the sequences SEQ ID NOs: 1-9 and 56;
(ii) the nucleotide sequence comprises a nucleotide sequence that is the reverse complement of a sequence as set forth in any one of the sequences SEQ ID NOs: 1-9 and 56;
(iii) the nucleotide sequence comprises the reverse complement of a sequence that is capable of hybridizing to the nucleotide sequence as set forth in (i) or (ii) under high stringency hybridization conditions or very high stringency hybridization conditions; and
(iv) the nucleotide sequence comprises a sequence having at least 90%, optionally at least 95%, preferably at least 97%, more preferably at least 98%, and most preferably at least 99% sequence identity to the nucleotide sequence as set forth in (i) or (ii).

7. The DEAR nucleic acid manipulation system of any of claims 1-6, wherein the target recognition site has a length of 6 nucleotides; and
preferably, the target recognition site is programmable to hybridize to a different target sequence.

8. The DEAR nucleic acid manipulation system of any of claims 1-7, wherein the target nucleic acid is DNA or RNA.

9. The DEAR nucleic acid manipulation system of any of claims 1-8, wherein the major cleavage site of the DEAR nucleic acid manipulation system is 0-1 nt downstream of the 3' end of the target sequence in the target nucleic acid.

10. An isolated polynucleotide, wherein the polynucleotide comprises a nucleotide sequence encoding the DEAR nucleic acid manipulation system of any of claims 1-9.

11. A nucleic acid construct, wherein the nucleic acid construct comprises the isolated polynucleotide of claim 10.

12. A vector, wherein the vector comprises the isolated polynucleotide of claim 10 or the nucleic acid construct of claim 11.

13. A cell, wherein the cell comprises the DEAR nucleic acid manipulation system of any of claims 1-9, the isolated polynucleotide of claim 10, the nucleic acid construct of claim 11, or the vector of claim 12.

14. A reagent or kit, wherein the reagent or kit comprises the DEAR nucleic acid manipulation system of any of claims 1-9, the isolated polynucleotide of claim 10, the nucleic acid construct of claim 11, the vector of claim 12, or the cell of claim 13.

15. A pharmaceutical composition, wherein the pharmaceutical composition comprises the DEAR nucleic acid manipulation system of any of claims 1-9, the isolated polynucleotide of claim 10, the nucleic acid construct of claim 11, the vector of claim 12, or the cell of claim 13; and optionally a pharmaceutically acceptable carrier.

16. A method for modifying a target nucleic acid, comprising the step of contacting the target nucleic acid with the DEAR nucleic acid manipulation system of any of claims 1-9, the isolated polynucleotide of claim 10, the nucleic acid construct of claim 11, the vector of claim 12, the cell of claim 13, or the reagent or kit of claim 14.

17. Use of the DEAR nucleic acid manipulation system of any of claims 1-9, the isolated polynucleotide of claim 10, the nucleic acid construct of claim 11, the vector of claim 12, or the cell of claim 13 in modifying a target nucleic acid or in preparing a reagent or kit for modifying a target nucleic acid.
